# EUROPEAN PATENT SPECIFICATION

(11) **EP 1 442 057 B1**
(45) Date of publication and mention of the grant of the patent: **13.06.2012**
(21) Application number: 02777580.8
(22) Date of filing: 24.10.2002
(51) Int. Cl.: C07K 14/47, C12N 5/10, A61K 48/00, C12N 15/12, C12N 15/86, C12N 15/867

(54) **USE OF A HALF-TRANSPORTER PROTEIN OF THE ABCG-FAMILY FOR SELECTING CELLS AND IN GENE THERAPY**
VERWENDUNG VON HALBTRANSPORTERPROTEINEN DER ABCG-FAMILIE ZUR ZELLSELEKTION UND IN DER GENTHERAPIE
UTILISATION D'UNE PROTEINE SEMI-TRANSPORTEUR DE LA FAMILLE ABCG DANS LA SELECTION DE CELLULES ET DANS LA THERAPIE GENIQUE

(30) Priority: 24.10.2001 HU 0104446; 04.03.2002 WO PCT/HU02/00015; 11.10.2002 HU 0203435
(43) Date of publication of application: 04.08.2004
(73) Proprietor: Creative Cell Kft., 1119 Budapest (HU)
(72) Inventor: NEMET, Katalin, H-1119 Budapest (HU); VARADI, György, H-1191 Budapest (HU); CERVENAK, Judit, H-2092 Budakeszi (HU); UJHELLY, Olga, H-1115 Budapest (HU); SARKADI, Balázs, H-1121 Budapest (HU); VARADI, András, H-1026 Budapest (HU); ÖZVEGY, Csilla, H-1123 Budapest (HU)
(74) Representative: Svingor, Adam
(86) International application number: PCT/HU2002/000108
(87) International publication number: WO 2003/035685

(56) References cited:
- WO-A-00/36101
- WO-A-02/071073
- EFFERTH T: "The human ATP-binding cassette transporter genes: from the bench to the bedside" CURRENT MOLECULAR MEDICINE, BENTHAM SCIENCE PUBLISHERS, GB, vol. 1, no. 1, March 2001 (2001-03), pages 45-65, XP001099044 ISSN: 1566-5240
- ZHOU S ET AL: "THE ABC TRANSPORTER BCRP1/ABCG2 IS EXPRESSED IN A WIDE VARIETY OF STEM CELLS AND IS A MOLECULAR DETERMINANT OF THE SIDE-POPULATION PHENOTYPE" NATURE MEDICINE, NATURE PUBLISHING, CO, US, vol. 7, no. 9, September 2001 (2001-09), pages 1028-1034, XP001098427 ISSN: 1078-8956
- HONJO Y ET AL: "Acquired mutations in the MXR/BCRP/ABCP gene alter substrate specificity in MXR/BCRP/ABCP-overexpressing cells." CANCER RESEARCH. UNITED STATES 15 SEP 2001, vol. 61, no. 18, 15 September 2001 (2001-09-15), pages 6635-6639, XP002228052 ISSN: 0008-5472
- DOYLE L A ET AL: "A MULTIDRUG RESISTANCE TRANSPORTER FROM HUMAN MCF-7 BREAST CANCER CELLS" PROCEEDINGS OF THE NATIONAL ACADEMY OF SCIENCES OF USA, NATIONAL ACADEMY OF SCIENCE. WASHINGTON, US, vol. 95, December 1998 (1998-12), pages 15665-15670, XP001055044 ISSN: 0027-8424
- KIM M ET AL: "THE MULTIDRUG RESISTANCE TRANSPORTER ABCG2 (BREAT CANCER RESISTANCE PROTEIN 1) EFFLUXES HOECHST 33342 AND IS OVEREXPRESSED IN HEMATOPOIETIC STEM CELLS" CLINICAL CANCER RESEARCH, THE AMERICAN ASSOCIATION FOR CANCER RESEARCH, US, vol. 8, no. 1, January 2002 (2002-01), pages 22-28, XP001094071 ISSN: 1078-0432
- KOWALSKI PETRA ET AL: "Modulation of the atypical multidrug-resistant phenotype by a hammerhead ribozyme directed against the ABC transporter BCRP/MXR/ABCG2." CANCER GENE THERAPY, vol. 9, no. 7, July 2002 (2002-07), pages 579-586, XP002228053 July, 2002 ISSN: 0929-1903
- WEIL WAYNE M ET AL: "Genetic correlation of p67-phox deficient chronic granulomatous disease using peripheral blood progenitor cells as a target for retrovirus mediated gene transfer." BLOOD, vol. 89, no. 5, 1997, pages 1754-1761, XP002228054 ISSN: 0006-4971
- SAROJ VELAMAKANNI ET AL: "ABCG transporters: structure, substrate specificities and physiological roles; A Brief Overview", JOURNAL OF BIOENERGETICS AND BIOMEMBRANES, KLUWER ACADEMIC PUBLISHERS-PLENUM PUBLISHERS, NE, vol. 39, no. 5-6, 8 November 2007 (2007-11-08), pages 465-471, XP019575817, ISSN: 1573-6881

## Description

The invention relates to uses of polynucleotides encoding an ABCG2 half-transporter protein of the ABCG-family as a selectable marker for selecting mammalian cells in vitro by drugs transportable by the transporter protein. The invention also relates to a vector for use in gene therapy, said vector useful for gene transfer into mammalian cells, and a pharmaceutical kit for use in gene therapy, said kit comprising the vector of the invention.

The invention relates to the field of selecting mammalian cells and of gene therapy.

A gene therapy process is an assembly of methods which may be suitable for treatment of diseases by use of new genes transferred into somatic cells. The range of potentially curable diseases is very broad. Hereditary diseases, caused by a deficiency of one or genes, comprise one large group of such diseases. The needed protein in most cases is not transcribed from these defective genes or the protein, though transcribed, is defective which leads to the clinical picture in question. In these cases the key step in the cure is the introduction of the missing intact or wild-type gene (cDNA).

A further domain of disorders is that of acquired diseases, wherein introduction of cDNAs encoding proteins not or only barely produced before opens up new vistas in medical sciences. In both cases treatment is based on the desired, newly produced protein.

When a cell is chosen for gene manipulation at least two important questions arise. First, whether a permanent or transient effect is to be elicited by the therapy and, second, what type of cells are touched by the disease. The target cell of the gene therapy can be any diseased or damaged cell. Nevertheless, an organism's various stem cells, being capable of reinstating a variety of tissues or organs, are especially suitable for gene therapy. Two basic forms of gene therapy are based on *in vivo* and *ex vivo* gene transfer, respectively.

Despite of general knowledge of the above principles and methods in the art, an important known obstacle of the application of gene therapy methods in the practice is the fact that the number of treatable cells relative to the number of all somatic cells is very small and, in addition, only a low ratio of the treated cells retains the required function. Moreover, the duplication rate of the manipulated cells in many cases lags behind that of the non-treated cells, and the curing effect of the treated cells is less than desired. Despite attempts in the art to increase the ratio of the effective cells the problem is unsolved (Licht et al., 1997).

It was suggested that the co-expression of a human drug-resistance protein with a therapeutic gene product in *ex vivo* modified stem cells should allow both an early enrichment of the corrected cells and an *in vivo* drug selection during clinical gene therapy. The use of the MDR1-P-glycoprotein as a selectable marker in gene therapy has already been investigated and advocated (Licht et al., 2000), (Bunting et al., 2000; Hafkemeyer et al., 2000; Schiedlmeier et al., 2002). However, in some experiments MDR1 expression rapidly declined in the modified cells (Sellers et al., 2001; Bunting et al., 2000). In other studies, although the expression level of MDR1 remained high, this caused alterations in stem cell maturation. The gene of this protein is well detectable in more than 50% of the human tumours, and is expressed in several normal tissues and a number of tumours. Thus, anti cancer drug treatment may induce expression of the protein. Moreover, the MDR1 protein may become produced in cells not comprising
MDR1 cDNA as a transgene, due to a drug treatment used to enrich transgene positive cells of a healthy patient. This, of course, decreases efficiency of selection. A further, major danger is, the malignant transformation of MDR1 expressing cells, which was experienced in some cases (Hafkemeyer et al, 2000) (Smeets et al., 1999).

Also, MDR1, a 170 kDa protein, has a large, over 4 kbase cDNA, making the construction of therapeutic vectors difficult. Moreover, the introduction of normal human MDR1 with a naturally wide drug substrate recognition does not allow a targeted selection of the genetically modified cells.

Thus, there is still a need in the art for a relatively simple and reliable system allowing selection and enrichment of successfully transformed cells.

The present inventors unexpectedly found that ABCG2, a multidrug resistance protein of the half-transporters of the ABCG2-family, can selectively protect cells from the effect of drugs used in chemotherapy, without many disadvantages of the prior art. This finding allows the first medical use of a protein family the members of which directly antagonize chemotherapeutic treatment in cancerous cells and their possible use has been seen in the development of inhibitors only (WO99/40110, WO01/36477).

### BRIEF DESCRIPTION OF THE INVENTION

The invention relates to the use of an isolated nucleic acid comprising a sequence encoding an ABCG2 half transporter protein of the ABCG-family for selecting somatic mammalian cells in vitro by at least one drug transportable by the transporter protein provided that cells of human embryonic origin are excluded. Thus, the isolated nucleic acid can be used as a selectable marker.

Said isolated nucleic acid is preferably a cDNA.

Preferably, the somatic mammalian cells are capable of proliferation or inducible to proliferate. The mammalian cells are preferably
- somatic pluripotent stem cells, bone marrow cells or hematopoietic progenitor cells, for example CD34+-cells.
- myeloid or lymphoid cells, for example *monocytes, dendritic cells,* T-cells, B-cells or NK-cells

The cells can be cells of an experimental animal, e.g. mouse cells, e.g. mouse bone marrow cells.

In a highly preferred embodiment, the ABCG2 variant is the R482G or the R482T mutant of the sequence deposited at GeneBank accession number AF093771

The nucleic acid is preferably carried by a vector capable of transfecting mammalian cells, e.g. a retroviral vector, preferably an oncovirus vector or a lentivirus vector.

The invention also relates to an isolated nucleic acid having a nucleotide sequence encoding an ABCG2 half-transporter protein of the ABCG-family for use in gene therapy, preferably, a nucleic acid for use in gene therapy by transforming mammalian cells with a vector carrying the isolated nucleic acid, expressing the half-transporter protein and selecting the cells by a cytotoxic drug which is transportable by said protein..

The isolated nucleic acid can be used in a treatment by a transgene which is a therapeutic gene, wherein the cells carrying the transgene and the isolated nucleic acid are protected by the expressed half-transporter protein against a cytotoxic drug transportable by the ABCG2 half transporter protein, or selected by the cytotoxic drug thereby the efficiency of said treatment by transgene is improved by the use of the nucleic acid. There are many diseases treating of which can be effectively improved by the method of the invention, preferably monogeneic, hereditary disorders, e.g. Gauchet-disease, beta-thalassemia, cystic fibrosis, hemophilia, muscular dystrophy, etc. In a preferred embodiment the disease -chronic granulomatous disease (CGD) and the therapeutic gene is gp91^{phox}.

Further disease types in which the invention can be applicable can be found in the publication and on the home page of (http://www4.od.nih.gov/oba/) NIH Office of Biotechnology Activities ("Recombinanat DNA and Gene Transfer") and in the Journal of Gene Medicine (Gene Therapy Clinical Trials) and on its home page (http://www.wiley.co.uk/wileychi/genmed/clinical/).

Furthermore, the isolated nucleic acid can be used in a gene therapy intervention in diseases treated by a cytotoxic drug, preferably during or accompanying chemotherapy of tumorous diseases.

The nucleic acid encodes an ABCG2 protein, more preferably an R482G or the R482T mutant of an ABCG2 protein preferably of the wild type human ABCG2 protein.

In a further aspect the invention relates to an expression vector for use in gene therapy, said vector being suitable for gene transfer into a mammalian cell, said vector comprising a nucleic acid sequence encoding ABCG2, operably linked to a promoter driving expression in a mammalian cell.

In a further embodiment, the vector is suitable for transient transformation of a mammalian cell.

Preferably, the vector is a viral vector, more preferably a retroviral vector.

In a preferred embodiment the vector of the invention comprises, besides the nucleotide sequence encoding the half-transporter protein, a further nucleotide sequence comprising the sequence of a transgene, e.g. an other therapeutic gene, i.e. the vector is preferably a bicistronic vector.

The vector of the invention preferably comprises a promoter operable in a mammalian cell, and preferably comprises means for ensuring that expression of the two nucleotide sequences is operably linked, i.e. that one can not be expressed without the expression of the other. Preferably, the therapeutic gene is located in a *cis* position, the nucleotide sequence encoding the half-transporter protein in a *trans* position relative to the promoter and between the two there is an SA site or an IRES site.

In a further embodiment, the retroviral vector is suitable for gene transfer into a mammalian cell unable to proliferate. Such vectors are e.g. lentivirus vectors.

In a preferred embodiment the mammalian cells to be transformed are those defined above.

The invention also relates to a somatic mammalian cell transformed by any of the vectors of the invention. In a preferred embodiment the cell is stably transfected by a viral, preferably a retroviral vector. In a further embodiment the cell is transiently transformed by any vector suitable for transformation of mammalian cells.

The invention also relates to an infectious virion, obtainable by a viral vector of the invention, a pharmaceutical kit and a pharmaceutical composition comprising a vector of the invention.

In a preferred embodiment the pharmaceutical kit comprises a viral vector and said kit optionally comprises one or more of the following: packaging cells applicable the viral vector, means for further viral gene manipulation, buffers, media, cell lines and reagents.

In a further aspect the invention relates to a process for protecting somatic mammalian cells against a cytotoxic drug transportable by an ABCG2 half transporter of the ABCG-family, said method comprising the steps of
i) treating the mammalian cells with a vector of the invention and thereby introducing a polynucleotide having a nucleotide sequence encoding a half-transporter protein of the ABCG-family into the cells,
ii) providing conditions appropriate for expression of the protein,
iii) exposing the mammalian cells to the cytotoxic drug and,
iv) testing the cells for resistance for the drug

In a preferred embodiment step iv) of the above process comprises selecting cells by the cytotoxic drug. Thereby the enrichment of the cell population in successfully transformed cells is possible. This preferred embodiment of the process is suitable for selecting mammalian cells, e.g. mammalian cells transformed by a transgene, using the nucleotide sequence encoding a half transporter of the ABCG-family as a selectable marker.

The invention further relates to a vector for use in gene therapy for use in a gene transfer method wherein cells treated with a transgene are protected against a cytotoxic drug transportable by a half transporter of the ABCG-family said method comprising the steps of
i) obtaining somatic cells to be treated from a mammalian patient,
ii) treating the cells with a vector of the invention and thereby introducing a nucleotide sequence encoding a half-transporter of the ABCG-family into the cells,
iii) reintroducing the treated cells into the patient,
iv) administering the cytotoxic drug to the patient, and
if desired, before or after reintroducing the treated cells, selecting the cells by a further cytotoxic drug, which can be the same as or different from the drug administered in step iv), transportable by the half transporter protein.

In the process of the invention the mammalian cells are preferably stem cells, more preferably CD34+ cells or bone marrow cells and the cells are treated with a retrovirus of any of the invention. The mammal can be a human or an animal, e.g. an experimental animal.

### DEFINITIONS

"ABC transporters" are transporter proteins belonging to the ABC protein superfamily and are capable of, in their native, active, wild type form, extruding drugs from the cells expressing them. Herein, the term "ABC transporter" also covers mutant variants of the wild type proteins retaining at least one function of the wild type, even if lacking activity.

"Multidrug transporters" are capable of extruding multiple kind of drugs from the cells.

A "half transporter (protein) of the ABCG family" is an ABC transporter which is a product of any of the *abcg* genes [such proteins are disclosed e.g. on the web-site http://nutrigene.4t.com/humanabc.htm/ and in publications of O'Hare et al., 1984; Pepling and Mount, 1990; Dreesen et al., 1988; Tearle et al., 1989 or a mutant, variant or homologue thereof which retains the transporter function of the any wild type form of the protein, preferably an active mutant variant or homologue.

The terms "ABCG2 protein", "ABCG2" or "ABCG2 transporter protein" are used interchangeably and are meant as a half transporter of the ABCG family, more closely the ABCG/white subfamily, and which is a product of the *abcg2* gene or a mutant, variant or homologue thereof which retains at least one function of the any wild type form of the protein, preferably a mutant variant or homologue which is an active transporter. ABCG2 is also termed as MXR (Mitoxantrone Resistance protein, Miyake et al., 1999.), BCRP (Breast Cancer Resistance Protein, Doyle et al.,1998) or ABCP (placenta specific ABC transporter, Allikmets et al.,1998). In harmony with the ABC nomenclature, we use the name ABCG2 multidrug transporter throughout the description.

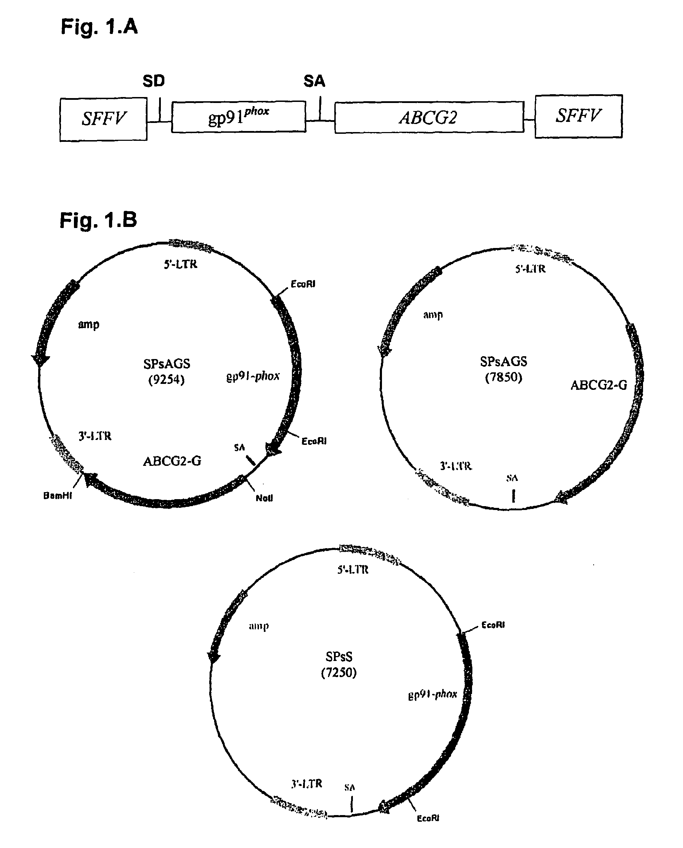

Members of the ABCG/white subfamily are ABC half-transporters with unique domain arrangement: they contain one ABC and one TMD where the ABC precedes (i.e. N-terminal to) the TMD (see Figure 1/B.). The product of the Drosophila white gene (a homologue of ABCG2) forms a heterodimer with the brown or scarlet ABC-half transporters and transports guanine or tryptophan, respectively [Pepling and Mount, 1990; Dreesen et al., 1988; Tearle et al., 1989]. Other identified members of the human white subfamily are ABCG1 (ABC8), ABCG5 and ABCG8 [Berge et al., 2000]. ABCG1 is involved in the cholesterol and phospholipid transport of macrophages [Klucken et al., 2000].

The sequence of the cDNA derived from the *abcg2* gene is available from several sources (WO 0136477, Doyle et al. (1998), and http://nutrigene.4t.com/humanabc.htm/). The Gene Bank accession number for the wild type cDNA encoding an R (Arg) at position 482 (ABCG2-R) is AF093771.

The term "isolated" is meant herein as "changed by man compared to its natural environment". If a compound or biological material, e.g. protein or its natural equivalent can be found in nature, than, if "isolated", then it is changed in its original environment or removed from its original environment or both.

A somatic cell is meant as a diploid cell, i.e. germ line cell are not covered by this term.

The term "capable of proliferation or inducible to proliferate" is meant as any cell capable to proliferate or which is possible to be made to proliferate by providing appropriate conditions, either in a living organism on in culture.

The term "stem cell" is meant as a somatic cell capable to differentiate in multiple directions. In a broad sense such cells are, starting from the cells of the zygote, each somatic cell capable of differentiation, e.g. cells of blastocyst, not entirely differentiated embrionic or fetal cells, to cells playing a role in hematopoiesis, e.g. cells capable to differentiate in many directions, e.g. SP-cells ("side population"; Zhou et al., 2001), CD34+ cells, pluripotent, myeloid and lymphoid stem cells etc.

The present invention does not aim at using human embryos themselves for industrial purposes or changing in any way the genetic material of human embryos. Thus cells of human embryonic origin are excluded from the scope of the claims.

The term "patient" may refer to either a human patient or an animal in need of treatment, preferably a mammal.

The rest of the terms is used in the description in the usual sense as understood by those skilled in the art considering, of course, the relevant context of the term in question.

Abbreviations are given as an endnote of the description. Other abbreviations, e.g. one-letter codes of amino acid, are used as common in the art.

Below the invention is described in more detail by illustrative examples. Publications referred to in the description, directly or indirectly, are incorporated herein by reference in their entirety.

### BRIEF DESCRIPTION OF THE FIGURES

Figure 1. A. Simplified illustration of the bicistronic SPsAGS vector used in this study. The gp91*^{phox}* cDNA is inserted behind an SFFV retroviral LTR and the splice donor (SD) site within the retroviral packaging site. The ABCG2-G cDNA (MXR) is inserted after a splice acceptor (SA) site derived from the genome of the Moloney murine leukemia virus.
   B) Schematic maps of the bicistronic SPsAGS vector, single cDNA SPsS vector used for mock transduction experiments and the monocistronic ABCG2 vector, SsAGS comprising cDNA coding for the R482G variant of ABCG2. Maps of vectors comprising other ABCG2 variants were the same.
Figure 2. Flow cytometry analysis of gp91*^{phox}* and ABCG2-G protein expression in transduced PLB985 X-CGD cells. Effect of mitoxantrone (MX) selection.
   Column 1: Mock-transduced cells; Column 2: transduced, unselected cells; Column 3: transduced, 10 nM MX selected cells.
   Panel A: Flow cytometry detection of gp91*^{phox}* expression (dotted lines: autofluorescence, solid lines: anti-gp91 mAb),
   Panel B: Flow cytometry detection of mitoxantrone uptake (dotted lines: autofluorescence, thick lines: MX fluorescence, thin lines: MX fluorescence in the presence of the ABCG2-inhibitor, FTC),
   Panel C: Immunocytochemistry demonstration of ABCG2-G protein expression. The red color (on black and white copies shown as shade-lines) indicates the anti-ABCG2 mAb staining, the cells are counterstained by the green fluorescent SYTO 24 nucleic acid stain.

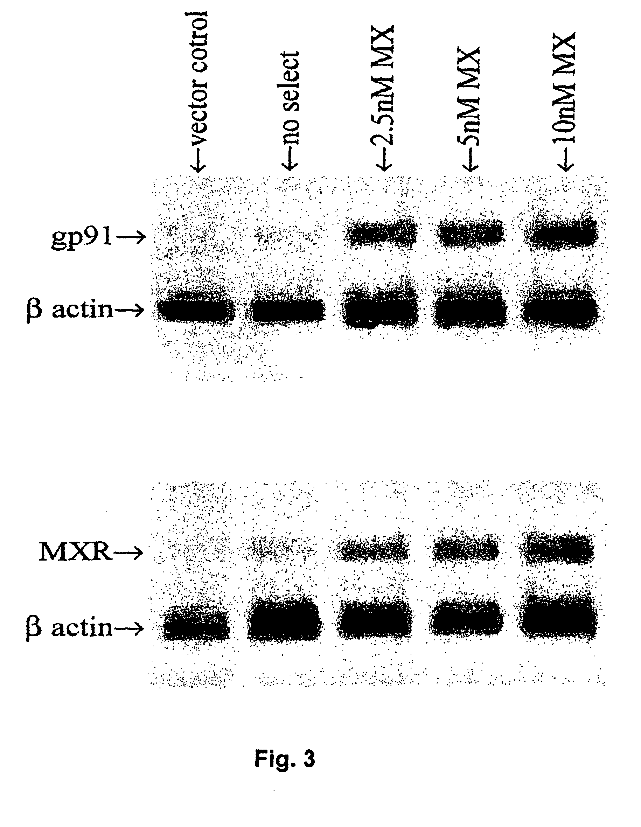

Figure 3. Semi quantitivequantitative PCR of the copy number of the SPsAGS vector from transduced CGD-PLB985 cells. ABCG2 and gp91^{phox} cDNAs were detected.

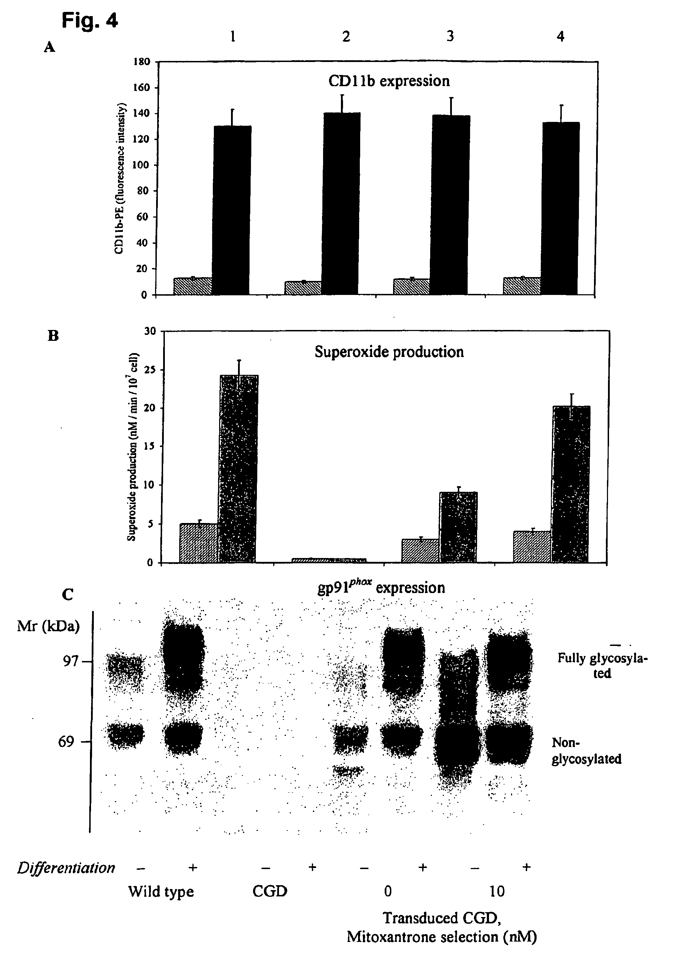

Figure 4. Effect of ABCG2-G protein expression and MX selection on the differentiation, superoxide production, and gp91*^{phox}* expression in PLB985 X-CGD cells.
   Column 1: Wild-type PLB985s; Colum 2: Mock-transduced PLB985 X-CGDs; Column 3: transduced, unselected PLB985 X-CGDs; Column 3: transduced, 10 nM MX selected PLB985 X-CGDs. In each column lane "-" represents the undifferentiated cells, while lane "+" indicates cells after 4 days of the induction of granulocyte differentiation.
   Panel A: Expression of the differentiation marker CD11b, detected by flow cytometry;
   Panel B: Superoxide production, detected by spectrophotometric cytochrome c reduction assay;
   Panel C: gp91*^{phox}* expression, detected by Western blotting (MoAb48).

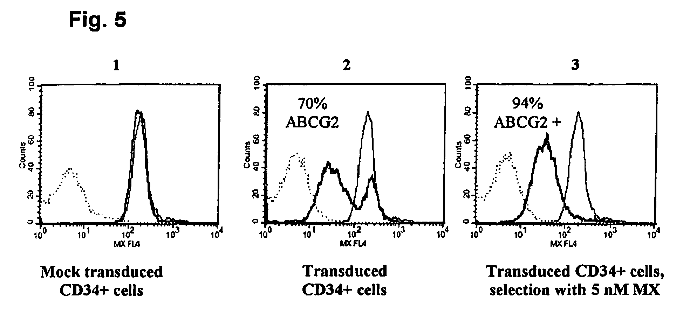

Figure 5. Effects of ABCG2-G protein expression and MX selection on mitoxantrone uptake in normal human CD34+ cells.
   Flow cytometry detection of mitoxantrone uptake (see Methods) in cells after gene transfer into CD34+ cells (dotted lines: autofluorescence, solid thick lines: MX fluorescence, solid thin lines: MX fluorescence in the presence of the ABCG2-inhibitor, FTC).
   Panel 1: Mock-transduced cells; Panel 2: transduced, unselected cells; Panel 3: transduced, 10 nM MX selected cells.

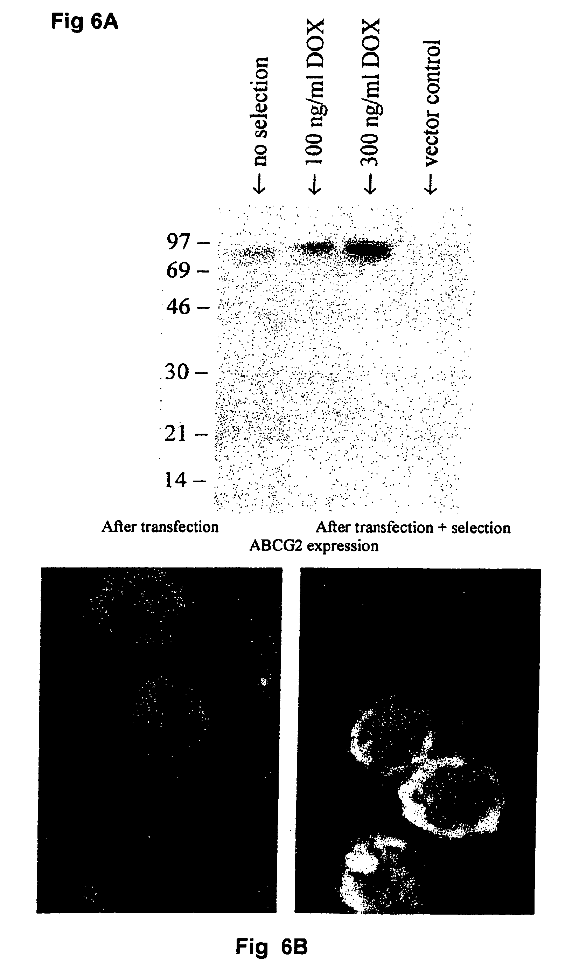

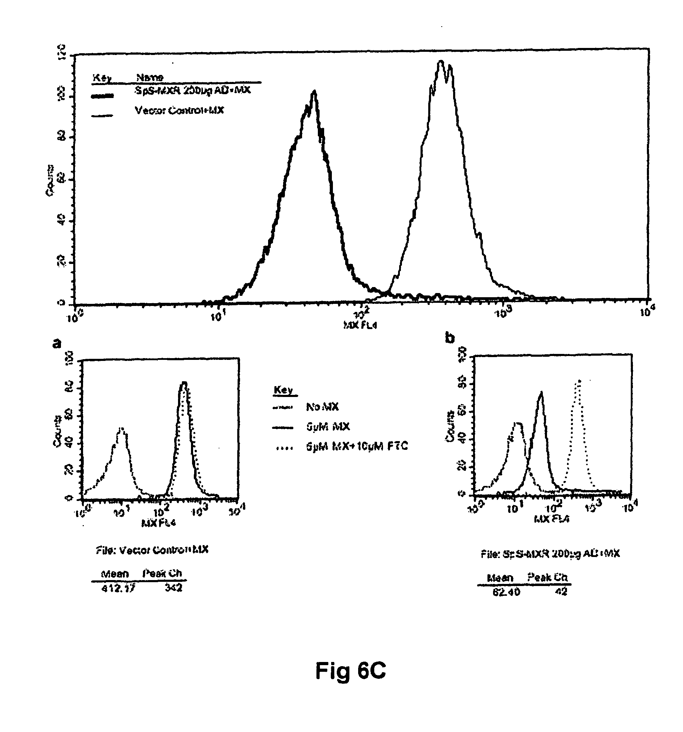

Figure 6. Western-blot (A) and immunochemical staining (B) of ABCG2 protein expressed in CGD-PLB985 cells transduced by SsAGS vector and selected on DOX. Figure 6 (C) shows MX-uptake measurements of CGD-PLB985 cells transduced by SsAGS vector and selected on DOX, detected by FACS.

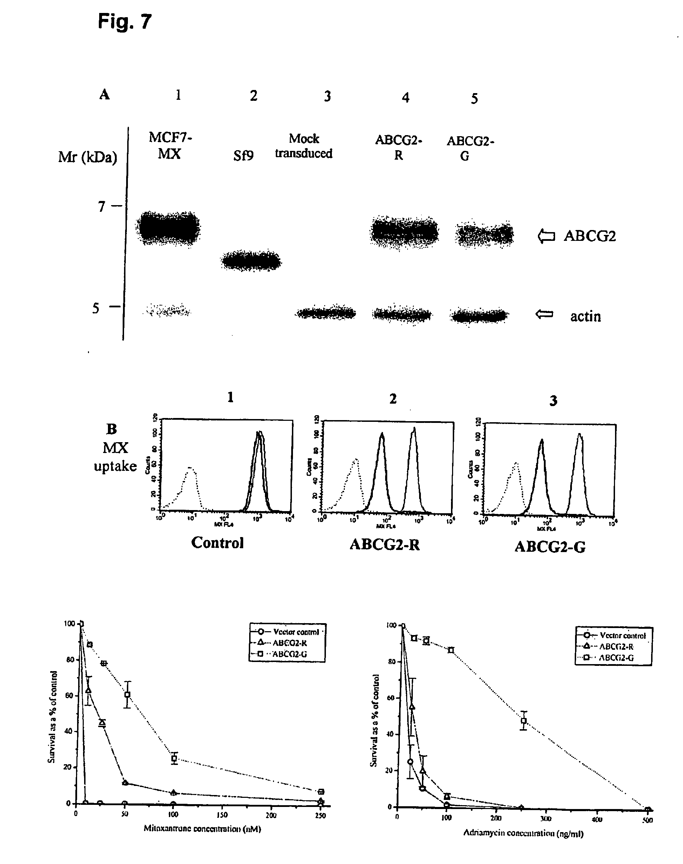

Figure 7. Expression of the ABCG2-G and ABCG2-R variants in PLB985 cells - effects on MX extrusion, adriamycin and MX resistance.
   Panel A: Expression of the ABCG2 variants, detected by Western blotting. The arrows indicate the ABCG2 protein (detected by the BXP-21 monoclonal antibody), and the cellular actin (detected by a polyclonal anti-actin antibody), respectively. Lane 1: MCF7-MXR cells, 1 µg protein, lane 2: Sf9cells expressing ABCG2, 0.3 µg protein, lane 3: Mock-transduced PLB985s, 25 µg protein, lane 4: PLB985s expressing ABCG2-R, selected by 10 nM MX, 25 µg protein, lane 5: PLB985s expressing ABCG2-G, selected by 100 nM MX, 25 µg protein.
   Panel B: Flow cytometry detection of mitoxantrone uptake (dotted lines: autofluorescence, solid thick lines: MX fluorescence, solid thin lines: MX fluorescence in the presence of the ABCG2-inhibitor, FTC). Part 1: Mock-transduced PLB985s; Part 2: PLB985s expressing ABCG2-R, selected by 10 nM MX; Part 3: PLB985s expressing ABCG2-G, selected by 100 nM MX.
   Panel C: Cytotoxicity assay (cell survival after 4 days) of PLB985 cells expressing the ABCG2 variants.
   ○ : Control, mock-transduced PLB985s;
   Δ : PLB985s expressing ABCG2-R, selected by 10 nM MX;
   □ : PLB985s expressing ABCG2-G, selected by 100 nM MX.

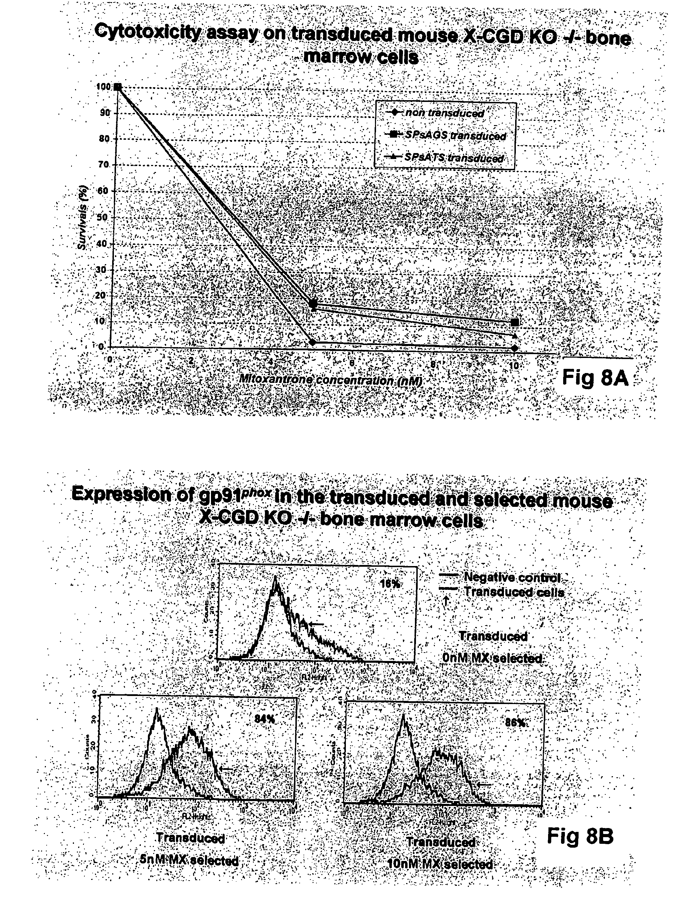

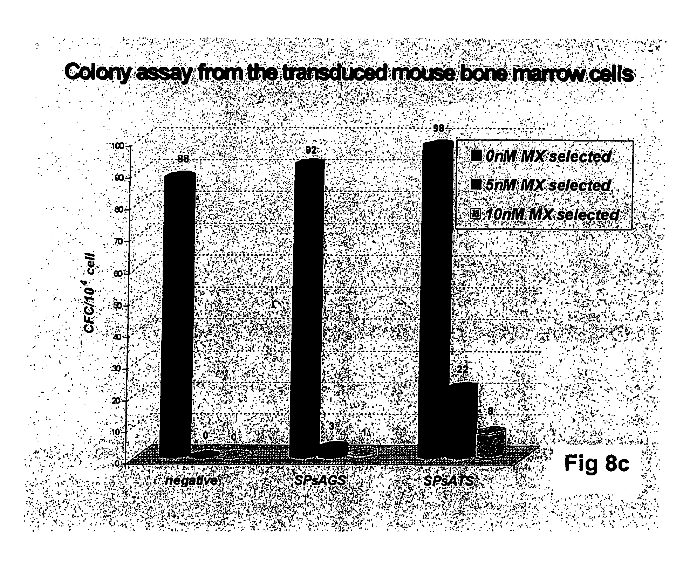

Figure 8. Panel A Cytotoxicity assay (cell survival after four days) on transduced mouse X-CGD KO -/- bone marrow cells
   ◆ Control, non transduced cells
   ■ Cells tranduced by SPsAGS vector carrying cDNA of ABCG2-G, unselected,
   ▲ Cells tranduced by SPsATS vector carrying cDNA of ABCG2-T, unselected.
   Panel B Expression of gp91^{phox} in the transduced and selected mouse bone marrow cells; (black line: negative control, gray line: transduced cell).
   Panel C: Colony assay from the transduced mouse bone marrow cells

### DETAILED DESCRIPTION OF THE INVENTION

The drug selection method of the invention can be applied in many fields. A common feature of all applications is the resistance of the cells expressing a half transporter of the ABCG2 family for drugs of interest.

Thus, the scope of cells transformable either *in vivo* or *in vitro* by the nucleotide sequence encoding the half transporter is broad: a cDNA coding for a member of the ABCG family, e.g. ABCG2, can be introduced in principle into any cell of an organism.

In the present invention, cells of any animal or human tissues can be applied. Preferably, stem cells or other blood cells are used.

Besides retroviral vectors, many vectors, actually any vector suitable for transforming mammalian cells and expression of foreign genes, can be applied: e.g. among viral vectors versions of adenoviruses, adeno-associated viruses, herpes or polioviruses, papilloma viruses manipulated for gene transfer. Gene transfer, however, can be achieved by non-viral methods which show a great variety. Such methods are e.g. the introduction of DNA conjugated to an other compound or to a liposome, or introduction of a naked vector. Gene transfer devices were also developed, e.g. gene gun (Robbins, 1997). Methods and devices for gene transfer are continuously improved and any method may prove to be suitable in the practice for introducing the gene applied in the invention.

Preferably retroviruses can be applied which facilitate stable incorporation of the transferred DNA into the genome of the host cell. Retroviruses used for gene therapy applications are preferably not able to propagate and have a very short life time, therefore are not dangerous for even patients without an immune defense. Such vectors are lentiviruses (HIV, FIV, SIV) adapted to gene transfer applications, by which not dividing cells and cells not inducible to proliferate can be transduced, and the introduced coding sequence is stably incorporated into the target cell genome. With lentivirus-based vectors in principle also resting nerve cells can be transfected, moreover stem cells, dendritic cells, etc (Saulnier et al., 2000; Schnell et al, 2001).

The majority of recent gene therapy experiments using modified retroviruses involves the basic elements of a mouse leukemia virus (MLV, "moloney murine leukemia"). Though a part of the proteins building up the virus particles is lacking or some of the coding sequences were replaced by proteins of other viruses, a common feature of these viruses is that they can transfect only propagating cells and stable incorporation of the introduced DNA into the target genome is ensured in principle (Baum et al., 1995).

According to a preferred embodiment of the invention a gene therapy method is provided or developed further *via* gene transfer into somatic mammalian cells by selecting the mammalian cells. Selection is carried out by expressing ABCG2 in mammalian cells after transduction and cells are exposed to a drug transportable by ABCG2. Thereby ABCG2 selectively protects cells from the effect of the drugs detrimental to the cells (toxic effect). As mentioned above, no suggestion in the art could be found for using ABCG2 as a selectable marker or for protecting cells in gene therapy.

To our knowledge the only successful retroviral gene therapy process resulting in a prolonged healthy state was carried out in a hereditary immune deficiency disease (SCID), which is caused by the fact that certain immune cells die very fast or can not evolve at all. After substituting the damaged gene region for a healthy one, functionally active cells capable of propagating and differentiating can form only from the cured cells (Cavanazza-Calvo et al., 2001). Thus, no separate selection step is required in the case ofthis disease.

For gene therapy application of ABCG2 we selected a severe inherited immunodeficiency, the chronic granulomatous disease (CGD). CGD in most cases is caused by mutations in the X-linked gene encoding gp91*^{phox}*, and by the consequent absence of a functioning superoxide-generating NADPH oxidase in phagocytes (Roos et al., 1996). This loss-of-function phenotype results in recurrent bacterial and fungal infections, leading to early death of the patients. Hematopoietic progenitor cell based gene therapy is a promising approach in CGD, as data from heterozygote carriers indicate that the presence of 10% normal phagocytes may be sufficient for the correction of symptoms (Malech, 1999). Still, so far only a limited success of clinical CGD gene therapy has been reported (Sokolic et al., 1996; Malech, 1999).

The therapy of the patients is presently symptomatic treatment. Bacterial or fungal infections in the organism, mainly in parenchimal organs (liver, lung) can be treated with antibiotics. Inside granulomas and abscesses emerging due to the disease living pathogens accumulate. However, the phagocytes crowding to the site can not kill the pathogens. Causal therapy of the disease is not solved.

Studies of carriers of the mutation revealed that if 10 to 15% functional cells occur among the non-functional phagocytes, asymptomatic state can be reached (Malech et al., 1997). Thus, this disease is an ideal target for gene therapy.

A mentioned, narrower substrate specifity of ABCG2 is advantage over MDR1. In this respect it would be rewarding to create mutants with an altered substrate specifity, methods for which are known according to the art. Uses of these mutant ABCG2-proteins in the invention are contemplated. Structural differences may give rise to differences in drug selectivity as exemplified in the Examples by mutants R482G and R482T. This may be utilized in selecting cells expressing different ABCG2 variants, or differentiating from the wild type variant.

Moreover, a person skilled in the art understands that sequences of further *abcg2* genes (or their cDNA) from further sources will be published in the future and mutant versions will be prepared. These sequences may also be appropriate for the purpose of the subject invention, provided that their necessary functions are retained, which can be decided readily by a skilled person based on the present disclosure.

From those explained above and from the examples the skilled person will understand that a selection provided by the half-transporters of the ABCG-family can be utilized in case of any therapeutic gene, if said therapeutic gene is transferred into the cell to be treated. The gene therapy method of the invention can be used in principle as a helper method in the gene therapy of any, acquired or hereditary disease based on dysfunction or lack of function of any gene or genes. Those diseases in the etiology of which an abnormal function of a blood cell plays a role, can be treated analogously to the methods described in the Examples. There are many surveys worldwide to provide a supplementary gene therapy for various diseases. As an example Gaucher-disease can be mentioned, which is an often serious, monogeneic hereditary disease caused by the lack of a lysosomal enzyme. Recently the therapy consists of the administration of the lacking enzyme which has to be continued lifelong and on the one hand is very expensive, on the other hand causes serious loss in the quality of life (Havenga et al., 1998). A similar possibility arises in respect of symptoms resulting from disorders of hemoglobin formation, like beta-thalassemia etc. (Havenga et al., 1998). Similarly to the present solution promising success in treatment of CGD, *in vitro* enrichment of cells expressing the transgene in necessary in these therapies, as well.

As further diseases e.g. cystic fibrosis, hemophilia, muscular dystrophy, etc could be mentioned or diseases given on the home pages of NIH and the Journal of Gene Medicine given above.

A further application field of the invention is the protection of the patient's cells from cytotoxic or chemotherapeutic drugs, whereby the therapeutic dose can be significantly increased. A main tool for treating tumorous diseases nowadays is chemotherapy, the application of which is limited by the drug's toxic effect to hematopoiesis. Therefore in the examples a method is taught which is useful to protect bone marrow cells of the patient. As will be understood all the cells in which protein of the ABCG family, e.g. ABCG2 can be expressed, e.g. retrovirally, can be protected against a drug transportable by the protein, provided that the cells can be reintroduced into the organism. A variety of the methods for retroviral transduction and reintroduction of cells is known according to the art (Williams and Smith, 2000).

If necessary, tumor cells shall be obliterated by *in vitro* purification so as not to become resistant.

The selection method of the invention is useful not only for therapeutic purpose. Selection of mammalian cells is needed in many cases in the laboratory and in the industry. Using retroviruses capable of selectively transducing certain cells and a posterior selection of these cells isolation of the cells becomes possible. Advantageous expression systems can be created if the cells expressing the desired protein can be selected.

On the basis of the present teaching vectors, e.g. retroviral vectors of the invention can be created from any retroviral vector which is suitable for transducing (depending on the virus or gene delivery vector) mammalian cells capable or not capable of proliferation.

### EXAMPLES

### 1. MATERIALS AND METHODS

### Cell lines and CD34+ cells

Phoenix-eco (Kinsella and Nolan, 1996) was a kind gift of G. Nolan, PG13 (Miller et al., 1991), retrovirus producing cells and 293 cells were obtained from the American Type Culture Collection, (Rockville, MD, USA). The human myelomonoblastic leukemia cell line PLB985 (Tucker et al., 1987) and its gp91*^{phox}*-knock-out variant (PLB985 X-CGD) (Zhen et al., 1993), were kindly provided by Dr. M. Dinauer. CD34+ cells were separated from cord blood samples (obtained with an informed consent). Mononuclear cells were isolated on Ficoll gradient, followed by Dynal magnetic separation (Dynal, Oslo, Norway). CD34+ enriched cell fraction was expanded in serum-free medium (X-Vivo 10 medium, Biowhittacker), containing a cytokine mixture (50 ng/ml SCF, 50 ng/ml IL-3, 1 ng/ml IL-6 and 50 ng/ml Flt3-ligand, Boehringer Mannheim Biochem, Germany) for 48 hours before transduction.

As mouse bone marrow cells Mouse X-CGD KO -/- bone marrow (BM) (RBC lysis, non-adherent fraction) were used.

### Vectors

The bicistronic SPsAGS vector (earlier name: SPsgp91MXR or SpS-91-MXR) was constructed by using the SPsLdS (Becker et al., 1998) vector. This bicistronic vector carries the gp91*^{phox}* cDNA inserted behind an SFFV retroviral promoter, while the ABCG2-G cDNA was placed 3' to the gp91*^{phox}* cDNA, after a murine leukemia virus splice acceptor-site (see Fig. 1). The NotI - BamHI fragment from the coding sequence of the R482G mutant MXR cDNA was used to replace the previously used LNGFR cDNA. Analogously, SPsATS containing the coding sequences for gp91*^{phox}* and R482T mutant of MXR/BCRP/ABCG2 constructed from SPsLdS was prepared. For mock transduction experiments, a single cDNA SPsS vector (earlier name: SPsgp91 or SpS-91) was constructed by removing the LNGFR cDNA. (Cutting out the NotI-BamHI fragment, creating blunt ends by T4 polymerase and ligating them.

In a monocistronic ABCG2 vector, LNGFR cDNA was replaced by the cDNAs coding for the R482 (SsARS) and R482G (SsAGS, earlier name: SPsMXR) variants of ABCG2, and the gp91*^{phox}* cDNA was also removed.

### Mutant cDNAs

Additional ABCG2 variants (482R, T) were created by overlap extension PCR [Horton, R.M. (1993) "In vitro recombination and mutagenesis of DNA SOEing together tailor-made genes" pp. 251-268]. In each case two internal complementary primer pairs were used, each containing the specific mutation: the primer pairs used for 482 R were 5' ttattaccaatgcgcatgttaccand and 5' ggtaacatgcgcattggtaataa, the primer pairs used for 482 T mutant were 5' ttattacctatgacgatgttacc and 5' ggtaacatcgtcataggtaataa. The two outer primer pairs used for the 482 variants were 5' cttgggatacttgaatcagc and 5' ggtcatgagaagtgttgcta. The PCR reactions were performed as described in Szakács et al., (2001). The PCR products containing the 482R or 482T coding sequence were digested with PstI and MscI enzymes and ligated between the corresponding sites of the pAcUW21-L/ABCG2 vector. The mutations were confirmed by sequencing the PstI-MscI or the NotI-SpeI fragments of the contstruct, respectively.

### Generation of retroviral particles

The Phoenix-eco packaging cell line was transfected by calcium phosphate co-precipitation. 48 hours after transfection the cell-free supernatant was collected and used immediately to transduce the PG13 packaging cell line (Becker et al., 1998). Single-cell clones were produced by limiting dilutions from cells containing the SPsAGS and SPsS vectors. The recombinant virus titer was tested by using 293T cells. For normal CD34+ cell transduction the retrovirus supernatant was concentrated by centrifugation at 180,000 x g for 1 hour.

Virus production for mouse bone marrow cells Phoenix-eco packing cells were transfected with calcium phosphate co-precipitation. 48-72 hours after transfection the cell-free supernatant was collected and frozen at -80°C until use. Viral titer was determined on NIH3T3 cells.

### Transduction of PLB985, PLB985 X-CGD and normal CD34+cells

Cells were transduced by the supernatant of the PG13 clones producing the highest titer virus. Transduction was performed in cell suspension, by low-speed centrifugation (spinoculation, 1,000 x g for 90 min, at ambient temperature) of 10⁵ cells with 1 ml viral supernatant, containing 1-2 x 10⁵ virus particles, in the presence of 8 µg/ml polybrene (in early experiments: 6 µg/ml). After centrifugation the cells were incubated at 32°C for 24 hours and a further 72 hours at 37°C with the viral supernatant and polybrene (in early experiments for 48 - 48 hours).

### Transduction of normal (C57B1/6 mice) or gp91^{phox}- knock out mice (KO-/-) bone marrow (BM) cells

BM cells were obtained from femurs and tibias of 6- to 8 week-old mice. After red blood cell lysis, total cell suspension was depleted from adherent cells and suspended in a cytokine mixture of murine IL-3 (10ng/ml), IL-6 (50ng/ml) and stem cell factor (SCF)(50ng/ml). After 2 days expansion cells were transduced twice by spinoculation (1,000 x g for 90 min, at 32°C).

### Assessing copy number of vectors semi-quantitative PCR

The presence of the vector in the cells and the increase of its copy number was followed by a semi-quantitive PCR method. PCR was carried out from the cells with gp91^{phox} and ABCG2 cDNA primers from DNA prepared by the *Gentra* kit. As a control, β-actin was used. The range where the rate of the production of the PCR-product shows a linear dependence from the cycle number was determined (Rolfs et al., 1992), and further measurements were performed after 25 cycle. The PCR-products were stained with SYBR Green I., run on agarose gels and the quantity of the obtained cDNA was estimated.

### Drug selection of the transduced cells

Transduced **PLB985** cells were selected by stepwise increases in mitoxantrone or doxorubicin (DOX; other name: adriamycin) concentrations. Selection was started 5 days (in some cases: 7 days) after transduction; each drug concentration was applied for four days (in early experiments: 8 days). CD34+ cells were selected similarly but were first selected on day two after transduction.

Selected cells were subjected to e.g. semi quantitative PCR, MX-uptake or gp91^{phox}-detection experiments.

### Immunodetection of gp91^{phox} and ABCG2 expression

Gp91*^{phox}* expression was measured by flow cytometry (Becton Dickinson FACS Calibur) by using the 7D5 antibody (provided by M. Nakamura (Nakamura et al., 1987) and phycoerythrin-labeled anti-mouse secondary antibody (GAM-PE, 250 times dilution, Sigma).

For immunoblotting (Western-blot) PLB985 cells were treated with 2 mM DFP and suspended in 2 x Laemmli buffer and sonicated for 5 seconds at 4°C. 10-25 µg protein/well was loaded on a Laemmli-type 7.5% SDS-polyacrylamide gel, the separated proteins blotted onto PVDF membranes, and immunodetection was performed as described previously (Muller et al., 1996) by using the monoclonal antibody BXP-21 (5,000 x dilution) for MXR (Maliepaard et al., 2001), and Moab 48 (200 x dilution) for gp91*^{phox}* (Verhoeven et al., 1989). The secondary antibody was an HRP-conjugated goat anti-mouse IgG (10,000 x dilution, Jackson Immunoresearch). Enhanced chemiluminescence (ECL) technique was applied to detect HRP activity on the blots. Quantitation of the ECL reaction was performed by using a Bio-Rad Phosphoimager.

For immunocytochemical detection of ABCG2, the cells were seeded onto glass microscope slides and fixed for 5 min at room temperature in 4 % paraformaldehyde in PBS. After 5 brief washes with PBS, the samples were further fixed and permeabilized in pre-chilled methanol for 5 min at -20°C. The cells were then blocked overnight at 4 °C in PBS, containing 2 mg/ml bovine serum albumin, 1 % fish gelatin, 0.1 % Triton-X 100, and 5 % goat serum. The samples were then incubated for 1 hr at room temperature with monoclonal anti-ABCG2 antibody, BXP-21 (Maliepaard et al., 2001), diluted 100 x in blocking buffer. After three washes in PBS, the cells were incubated for 1 hr at room temperature with Alexa-568 conjugated goat anti-mouse IgG (H+L), diluted 250 x in blocking buffer. After repeated washes, the samples were counterstained by SYTO 24, 200 nM, for 15 min at room temperature. Subsequently, the slides were studied on a RatioMaster-D video imaging system (Photon Technology, Inc), mounted on a Zeiss Axiovert 135 microscope using a Zeiss Fluar 100 x (1.3) oil immersion objective. For green and red fluorescence acquisitions the samples were illuminated with 480 and 570 nm excitation light (band widths 3 and 10 nm, respectively), and pairs of images were taken at 515-545 nm, and >590 nm, respectively. Images were imported and edited using Paint Shop Pro 7.04.

As an alternative possibility the method of Rocchi et al was used (Rocchi et al., 2000). In the present experiments this method was used after DOX selection of ABCG2-G expressing cells (figure 7.B). Cells to be studied were centrifuged onto slides, fixed in ethanol, then incubated in a solution of polyclonal anti-ABCG2 antibody, diluted 100 x, at 37°C at room temperature. Slides were washed in PBS, then reaction was detected with diluted FITC - anti-rabbit-IgG (Sigma). Cell preparations were evaluated by using a fluorescent microscope (Olympus BH-2).

### Mitoxantrone uptake

Functional detection of ABCG2 expression was evaluated with a modified MX uptake assay of Robey et al. (Robey et al., 2001). Cells were suspended in phenol red-free Hank's balanced salt solution, with additional pH stabilization by 20 mM phosphate buffer. Aliquots of the suspension, containing 3x10⁵ cells, were incubated with or without the addition of 5 µM MX, and also with 5 µM MX + 10 µM FTC, a specific inhibitor of ABCG2 (Rabindran et al., 2000; van Loevezijn et al., 2001). After one hour incubation at 37°C, the cells were washed with ice-cold medium, resuspended in ice-cold medium containing 2 g/ml propidium-iodide, and stored on ice until analysis by flow cytometry. Cellular MX values were determined at 635 nm excitation/ 661 nm emission wavelengths.

### Cytotoxicity assay

This assay was performed in 24-well plates, cells seeded at a density of 200,000 cells/well. Cytotoxic drugs were added at the concentrations indicated and the cells were cultured for 4 days at 37°C. Total and live cell numbers were determined by flow cytometry after the addition of 2 µg/ml propidium-iodide, to identify dead cells.

### Granulocyte maturation and measurement of superoxide and oxygen-radical production

Cell differentiation was induced by 0.5% dimethyl-formamide (DMF) in order to obtain granulocytes. In order to follow granulocyte differentiation, cells were labelled with monoclonal anti-CD11b/RPE antibody (Dako, Denmark), and staining was determined by flow cytometry. For the induction of differentiation in the case of normal CD34+ cells, both liquid suspension cultures and clonogenic progenitor assays were used. For suspension culture, fresh media, containing cytokines + G-CSF (50 ng/ml) were added to the cells 24 hours after transduction, and then the cells were incubated for 12-14 days at 37°C.

In the colony-forming cell assay the cells were plated at a concentration of 250 cells/ml and 1250 cells/ml in Iscove's medium, containing 0.9% methylcellulose, 30% FCS, 1% bovine serum albumin, 1.4 x 10⁻⁵M dithiothreitol, 5 ng/ml human recombinant IL-3, 25 ng/ml SCF, 10 ng/ml GM-CSF, 2 ng/ml G-CSF and 2U/ml erythropoietin, in the presence or absence of 5 nM mitoxantrone. After 14 days of incubation at 37°C, the colony forming cells were counted.

Superoxide production was determined by the superoxide dismutase (SOD) inhibitable reduction of cytochrome c (Babior et al., 1973), by incubating 2 x 10⁵ cells stimulated by 70 nM phorbol-12-myristate-13-acetate, and measuring ΔE₅₅₀ by a HP8451 diode array spectrophotometer.

The nitroblue tetrazolium (NBT) assay was used to measure the oxygen-radical formation at a single cell level. Cell suspensions were mixed with 80 nM phorbol-12-myristate-13-acetate + ^{~~}µ nitroblue tetrazolium (NBT) in HBSS, and incubated at 37°C for 1 hour on a glass slide. After ethanol fixation the cells were scored for the presence or absence of blue staining.

### Experiments with mouse bone marrow cells

Vector used for experiments with mouse bone marrow cells were the same as in the in vitro study.

Drug selection of the transduced cells, cytotoxic assay, immunodetection of gp91*^{phox}*, nitroblue tetrazolium test (NBT) were performed similar as in human CD34+ cells except using murine cytokines. In the colony assay 10 000 cells were plated in semisolid medium containing 0, 5 and 10nM MX.

### 2 RESULTS AND DISCUSSION

### Co-expression of gp91^{phox} and ABCG2 in progenitor cells - effect of drug selection

In order to produce recombinant retroviruses, a bicistronic retroviral vector was constructed, carrying the coding sequences for gp91*^{phox}*, and those for the normal ABCG2, or the R482G variant (denoted as ABCG2-G). The gp91*^{phox}* cDNA was inserted behind the SFFV retroviral long terminal repeat (LTR), while the ABCG2 cDNA was placed after the splice acceptor-site of murine leukemia virus (see Fig. 1). Retroviruses were produced by a pseudotyping (GALV-env) packaging system to yield a high and stable virus titer, and an efficient infection of the target cells (see Methods).

In order to follow a functional expression of the therapeutic gene, the recombinant retroviruses were used to transduce an X-CGD knock-out variant of a human promyelo-monocytic cell line, PLB985. The cells underwent a controlled drug selection by mitoxantrone (MX), which was followed by the induction of granulocyte maturation.

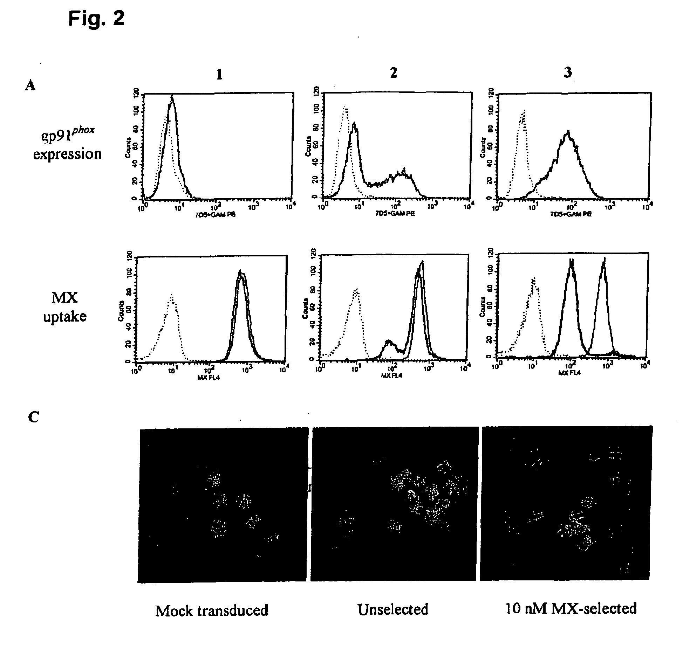

Fig. 2, Panel A, presents the expression of the gp91*^{phox}* protein in PLB985 X-CGD cells, as measured by flow cytometry. The anti-gp91*^{phox}* monoclonal antibody 7D5 recognizes the human gp91*^{phox}* on the cell surface (Yamauchi et al., 2001). As shown in Panel A1, PLB985 X-CGD cells did not express this protein, while 5 days after retroviral transduction the presence of 40-48 % gp91*^{phox}*-positive cells could be detected (Panel A2). When the cells were subjected to 10 nM MX selection for four days, the ratio of gp91*^{phox}*-expressing cells increased to over 90% (Panel A3), similar to that seen in wild-type PLB985 cells (data not shown).

In the same retrovirally transduced PLB985 X-CGD cells the function of the ABCG2-G protein was followed by cellular MX uptake experiments. The effect of a selective inhibitor of the ABCG2 function, fumitremorgin C (FTC), (Rabindran et al., 2000), was also examined. As documented in Panel B, MX uptake inversely correlated with gp91*^{phox}*-expression. The control, mock-transduced cells had a high level of MX uptake and there was no effect of FTC in these cells (Panel B1). In contrast, retrovirally transduced cells showed a double peak in the MX uptake curve: a large fraction of the cells showed high MX uptake, while in a relatively small fraction of the cells MX uptake was much smaller, and significantly increased by the addition of FTC (Panel B2). These low-MX-uptake cells became a uniform population after pre-selection with 10 nM MX, and MX uptake in these cells was fully sensitive to the addition of FTC (see Panel B3).

Fig 2, Panel C documents the expression of the ABCG2-G protein by immuno-cytochemistry. The cells were fixed, permeabilized, and stained for ABCG2 by the monoclonal antibody BXP-21, and a phycoerythrin-conjugated second antibody. Nuclear counterstaining was obtained by the green fluorescent SYTO 24. As shown, untransduced PLB985 cells (Panel C1) showed only a low level background staining for ABCG2. After the retroviral transduction (Panel C2) several cells had a high level expression of ABCG2. When mitoxantrone selection followed the transduction (Panel C3), high-level ABCG2 immunostaining was uniformly present in most of the cells.

These data demonstrate a successful co-expression of gp91*^{phox}* and ABCG2 in retrovirally-transduced PLB985 X-CGD cells, and a positive selection of the ABCG2 co-expressing cells by the cytotoxic agent, mitoxantrone. These data clearly show the occurrence of the splicing event during transcription of the bicistronic retroviral vector. It is important to note that the transduced and MX-selected cells could be cultured in drug-free media for several months without a significant loss of the high-level transgene expression of both gp91*^{phox}* and ABCG2 (data not shown).

### Vector copy number determination

The above findings are further supported by detection of the vector copy number by multiplex PCR (Figure 3). Due to drug (MX) selection the amount of both cDNA cloned into the SPsAGS vector, i.e. cDNAs of gp91^{phox} and ABCG2 was definitely increased, depending on the concentration of the drug during selection.

### Granulocyte maturation and functional evaluation of gp91^{phax} and ABCG2 expression

In the following experiments we studied the granulocyte maturation and the functional reconstitution of the NADPH enzyme activity in the human PLB985 X-CGD cells, after retroviral transduction and MX selection, as described above. PLB985 cell differentiation into granulocytes can be induced by dimethyl-formamide (DMF) and the differentiated cells express the CD11b adhesion protein as a marker of maturation. Wild-type PLB985 cells, after stimulation by phorbol-12-myristate-13-acetate (PMA) show a high-level superoxide production, reflecting the NADPH oxidase activity. An essential component of the superoxide-producing enzyme complex is gp91*^{phox},* which becomes highly glycosylated during granulocyte maturation (Hua et al., 2000). Both gp91*^{phox}* expression and superoxide production are practically absent in the PLB985 X-CGD cells.

Figure 4A documents CD11b expression in the wild-type or PLB985 X-CGD cells, before and after the induction of cell differentiation by DMF. As shown, this treatment caused a uniformly high CD11b expression both in the wild-type and the PLB985 X-CGD cells, even when these latter cells were expressing high levels of ABCG2, following 10 nM MX selection. Thus, granulocyte maturation seems to be unaltered by the overexpression of ABCG2.

Fig. 4B shows superoxide production in PLB985 X-CGDs upon the induction of granulocyte maturation and stimulation by PMA. We found that superoxide production was very low both in the undifferentiated wild-type PLB985 cells and PLB985 X-CGD cells. In the wild-type cells superoxide production increased to a high level after 5 days of granulocyte differentiation and stimulation by PMA. PLB985 X-CGD cells produced no superoxide under any condition. Retrovirally transduced PLB985 X-CGD cells, after differentiation and PMA stimulation, exhibited a significantly higher superoxide production, reaching 36-40% of the level seen in the wild type PLB985s. This superoxide production increased significantly, up to the level seen in the wild-type PLB985s, following MX selection of the transduced PLB985 X-CGDs.

The expression and maturation of the gp91*^{phox}* protein in retrovirally transduced and drug-selected PLB985-CGDs was also followed by gp91*^{phox}* immunoblotting (Fig. 4C). In these experiments the Western blots for the above PLB985 X-CGDs were developed by the MoAb48 antibody, reacting with an intracellular epitope of gp91*^{phox}* (Burritt et al., 2000). As shown, in contrast to the wild-type PLB985s, in the PLB985 X-CGDs no gp91*^{phox}* expression was observed, while this protein appeared in the transduced cells. Gp91*^{phox}* expression significantly increased following MX selection. This protein appeared only core-glycosylated in the immature cells (bands at 65-75kDa), while in differentiated cells a fully glycosylated gp91*^{phox}* could be observed, as a wide band between 91-180 kDa. These data indicate the proper reconstitution of the functional NADPH oxidase activity, based on gp91*^{phox}* expression and maturation, in the ABCG2-expressing cells.

In order to examine the possible effects of ABCG2 expression and drug selection on natural human progenitor cell differentiation, we carried out similar experiments by using normal human cord blood heamatopoietic progenitor (CD34+) cells, and the same vectors as above (Fig. 5). After retroviral transduction the cells were exposed to 5 nM MX selection for 3 days, and induced for granulocyte differentiation by G-CSF, or plated directly in colony forming cell assay and selected by MX in methylcellulose. Normal or mock-transduced CD34+ cells did not show any measurable MX extrusion capacity (Panel 1). In contrast, we observed a significant increase in MX extrusion in the transduced progenitor cells (Panel 2), and this MX extrusion was further increased by MX selection of the transduced cells (Panel 3).

As indicated by the data in Table I, both the mock-transduced, and the ABCG2-G transduced and MX selected cells properly differentiated into granulocytes. In both cases about 70-80% of the cells expressed CD15, the cell surface marker for granulocytes and monocytes. In the suspension cultures mock-transduced cells were eliminated by 5 nM MX selection, while in the ABCG2-G transduced cells an unchanged level of CD15+ cells was observed. In parallel experiments we found that a similar number of cells gave a positive NBT reaction after differentiation, both in the case of the mock-transduced and the ABCG2-G transduced cells (data not shown).

When measuring the number of colony forming cells (CFC), we found that this was not different in the mock-transduced and ABCG2-G transduced cells, respectively (see Table I). When 5 nM MX, as a selection agent, was included in this assay, the mock-transduced cells did not form colonies, while the MX selection decreased the CFC number in the ABCG2-G transduced cells by about 50%. This reduction closely corresponds to the elimination of the cells not expressing ABCG2-G (see Fig. 5). These experiments indicated no measurable effect of ABCG2 expression on the differentiation and colony-forming ability of human CD34+ progenitor cells.

### Studies after transduction with SsAGS; selection on DOX

Similar studies described above were performed after transduction of cells with the monocystronic SsAGS vector comprising cDNA of ABCG2-G alone. Expression of the protein was followed by Western-blot before and after drug selection. On figure 6, panel A it is shown that while from unselected cells disrupted after transduction no ABCG2 protein could be detected, cells cultured on 300 ng/ml DOX showed a significant expression of the transgene. DOX, a medicine used in chemotherapy, similarly to MX, is pumped out from the cells expressing ABCG2, whereas ABCG2-negative cells die. Thus selection was possible with multiple type of drugs.

In the selected cells immunohystochemical detection of ABCG2 could be achieved. Figure 6, panel B demonstrates the occurrance of ABCG2 in the membrane of the transduced cells. In harmony with the above Westem-blot results, the protein can be seen only in traces in the treated cells. A higher, well detectable quantity of the protein is produced in DOX-selected cells.

Data obtained by MX-uptake (figure 6, panel C) further supports the above conclusions. While fluorescence intensity of cells treated only with MX (in the case of vector control) is essentially the same as that of the MX + FTC comprising sample, cells pretreated with 300 ng/ml DOX show a significant shift in fluorescence, relative to FTC. Thus, the expression of ABCG2 inhibits MX uptake.

### Selective protection of the transduced cells against cytotoxic drugs by the mutant ABCG2 protein

In the above-described experiments we used an R482G variant of the ABCG2 protein as a selectable marker. In the established human genome sequence Arg is found at this position (Gene Bank accession number: AF093771) and our own studies have also shown that in the normal population only this variant could be detected (results to be published elsewhere). It has been recently reported that the R482G and R482T mutant variants of ABCG2 have altered substrate specificity. In ABCG2-expressing cancer cell lines (Honjo et al., 2001), and in human ABCG2 expressing Sf9 cells (Özvegy et al., 2002) an ATP-dependent extrusion of MX and the Hoechst 33342 dye was found in all variants, while Rhodamine 123 or ADR was not efficiently transported by the wild-type ABCG2 protein. In isolated membranes all three ABCG2 protein variants (482G, T and R) had a relatively high basal, vanadate-sensitive ATPase activity, and when measuring the uptake of fluorescent compounds in ABCG2-overexpressing intact Sf9 cells, we found that all the three 482 variants of ABCG2 acted as active extrusion pumps, inhibited by Fumitremorgin C (FTC). However, only the 482G and 482T mutants showed drug-stimulated ATPase activity, when measured in the presence of known ABCG2 substrates.

In order to investigate the function and expression of these variants, we generated cDNAs encoding both the wild-type and the R482G ABCG2 protein, and inserted each of these cDNAs into the retroviral vector presented above (see Fig. 1). Recombinant retroviruses were produced and used to transduce PLB cells as described above. Transduced cells were cultured in media containing 5-100 nM MX. The expression and function of the ABCG2 protein was followed by immuno-detection, and by measuring MX uptake and cytotoxicity.

As documented in Fig. 7, after retroviral transduction and selection for 4 days in increasing concentrations of MX in the media, PLB985 cells expressed both the wild-type and the R482G variant of ABCG2 in a functionally active form. The level of ABCG2 expression, determined by immunocytochemisty or by Western blotting, depended on the MX selection conditions applied. In order to provide comparable functional data, we examined PLB985 cells expressing similar amounts of the ABCG2 variants.

As shown in (Fig. 7, Panel A), and based on the quantitative evaluation of the Western blot lanes (using actin as an internal protein control), PLB985 cells transduced by the wild-type ABCG2 and selected in 10 nM MX (lane 4), expressed a similar (according to a Phospho-Imager evaluation, 1.6 times higher) level of the ABCG2 protein, then the corresponding cells expressing the R482G variant, and selected in 100 nM MX (lane 5). For comparison, we also present the expression of ABCG2 in mock-transduced PLB985 cells (lane 3), in MCF7-MX cells (lane 1) and in Sf9 cells (lane2). In the functional studies we compared these PLB985 cells for mitoxantrone uptake and cytotoxicity effects.

As documented in Fig. 7, Panel B, MX uptake was high in the mock-transduced cells, and significantly reduced in both ABCG2-R and ABCG2-G expressing PLB985 cells. MX extrusion was fully reversible in both cases by the addition of the ABCG2 inhibitor, FTC. In parallel studies we documented that FTC was about equally effective inhibitor for both ABCG2 variants (PCT/HU02/00015, Özvegy et al., 2002, see below). These findings indicate a similar, or somewhat greater MX transport activity for the ABCG2-G variant than for the wild-type protein.

In Fig. 7, Panels C and D document the cytotoxic effects of increasing concentrations of MX (Panel C) and DOX (the same as adriamycin, ADR Panel D), by using the same ABCG2-transduced PLB985 cells. As shown, the IC50 value for MX in the vector control transduced cells was about 10 ng/ml, while the presence of the ABCG2-R increased this value to 30 ng/ml, and that of the ABCG2-G variant to 75 ng/ml. These findings are in line with the MX extrusion data presented in panel B.

As shown in Panel D, the IC50 value for ADR in the control PLB985s (about 8 ng/ml) shifted dramatically (to about 250 ng/ml) in the PLB985 cells expressing ABCG2-G, while this value changed only slightly (to about 20 ng/ml) in the case of the wild-type ABCG2-R. Thus, with an actually somewhat higher expression level (see Panel A), the wild-type ABCG2 gave practically no protection against ADR. This specific drug-sensitivity profile strongly suggests that the ABCG2-G expressing progenitor cells can be efficiently selected e.g. by DOX (adriamycin), even in the presence of relatively high levels of the endogenous, wild-type ABCG2 protein.

### Experiments on cells of an animal model: mouse bone marrow cells

In order to investigate the expression of human gp91*^{phox}* in the transduced gp91*^{phox}*- knock out (KO-/-) mice bone marrow cells, the recombinant retroviruses produced by the ecotropic packaging cell line, Phoenix-eco were used. The envelope protein of these virus particles is ready to bind to rodent cells and infect them efficiently. The vector packaged in the viruses was equal with the bicistronic one for the human cells (SPsAGS) plus the same vector with the coding sequence for ABCG2-T (SPsATS) was applied instead of ABCG2-G . The transduced cells were checked for gp91*^{phox}* expression without drug selection or following 5 days of 5nM or 10nM mitoxantron selection.

Ratio of surviving cells after 4 days of drug selection was shown in Fig.8.A. Non-transduced and SPsAGS or SPsATS vector transduced gp91*^{phox}* knock out BM cells were treated with 5 or 10nM mitoxantron for 5 days. This treatment started 5 days after transduction without any previous selection of the cells. As shown in the figure, all non-transduced control cells died in 5nM or more mitoxantron, while about 15-20% of the transduced ones expressed resistance to MX. There was no marked difference between the two mutant forms of ABCG2.

Fig. 8.B, presents the expression of the gp91*^{phox}* protein as measured by flow cytometry followed by indirect labeling of the cells with 7D5 monoclonal antibody (similar as seen in Fig. 2. Panel A.). Cells were transduced with (SPsAGS) . Grey line in each panel shows the autofluorescence of the untransduced KO-/- mice bone marrow cells, while black line represents the fluorescence intensity of 7D5 labeled cells . It can be seen that a small increase in fluorescence appeared in transduced cells, while after 5 days selection on 5nM or 10nM MX, more then 80% gp91*^{phox}*-positive cells could be detected.

When measuring the number of colony forming cells (CFC), we found that this was not different in the non-transduced and ABCG2-G, or ABCG2-T transduced cells, respectively (Figure 8.C). When 5 nM or 10nM MX, as a selection agent, was mixed with the semisolid medium, the non-transduced cells did not form colonies, the ABCG2-G transduced cells hardly formed any, while MX selection decreased the CFC number in the ABCG2-T transduced cells by about 80%. This reduction closely corresponds to the elimination of the cells not expressing ABCG2-T.

Conclusion of the *in vitro* study with mouse BM cells: retroviral transfer of ABCG2 substrate mutants together with gp91*^{phox}* revealed that cells had a selective advantage over non-transduced ones, when exposed to mitoxantron. Although the transduction rate was very pure vector containing BM cells could be enriched by selection with MX, a useful chemotherapeutic agent. These experiments will be followed by in vivo studies in mice.

### An example for a gene therapy method

The gene therapy method described below is a supplementary therapy for autologous bone marrow transplantation. Bone marrow progenitor cells of the patient to be treated are induced to enter the blood stream, e.g. by a mild drug treatment (e.g. cytokines). Separation of progenitor stem cells can be carried out by using the CD34 marker, (see Materials and Methods, 1.1. Cell lines and CD34+ cells). This marker is carried by non differentiated cells. An antibody produced against this protein is bound to magnetic beads. White blood cells of the patient are mixed with the beads. Cells expressing CD34 bind to the magnetic beads and can be selected by a magnet. The rest of the cells which are not bound, are given back to the patient immediately after the process (leukapheresis, Ishizawa et al., 1993). After this gene manipulation is carried out on the purified CD34+ cells. A part of the separated cells are frozen under liquid nitrogen (for later use). Treatment, if retroviral vector is used, lasts for 2 or 3 days, during which period the cells are multiple time infected with the viruses carrying the cDNA of interest, while said cells are maintained in gas permeable sacks and cultured by adding and appropriate medium. After this treatment cells are administered to the patient as an injection. The healthy, cured cells are retained in the bone marrow where hematopoiesis is started from the modified cells. Many modified version of the above method is known for a skilled person (Berger et al., 2001, Havenga et al., 1998).

If control studies suggest that no sufficient number of cells, expressing the desired curing protein, entered the circulation, a short period treatment of the patient with a low dose cytotoxic agent may be started. Thereby those cells, which survive the cytostatic treatment, will be transgene positive with a high probability.

### Preclinical studies

The *in vivo* effect of ABCG2 variants can be used in the treatment of patients after gene therapy, when the ratio of gene-treated cells is low, or when a decrease in the trans-gene expressing cells occur. Preclinical studies will be performed in the near future to investigate the *in vivo* effect of the bicistronic retroviral vector used in the in vitro experiments so far. The *in vivo* animal model is planned to be mice, a draft version of the experimental protocols see below:
Murine stem cells from normal, gp91*^{phox}-* knock out or NOD-SCID mice can be transduced by retrovirus containing the previously mentioned vector. Bone marrow transplantation of lethally irradiated mice with the adequate autologue stem cells, pre-treated with retrovirally transduced cells may repopulate mouse bone marrow.

Selection potential of ABCG2 can be investigated even in a normal mice strain, while gp91*^{phox}*- knock out mice offers the additional possibility to show the reconstitution of NADPH oxidase function after transplantation.

A NOD SCID mice model provides a pre clinical study, which is useful for several reasons: bone marrow transplantation of a mice can be performed using gene-treated human stem cells. In this special animal strain, because of the serious immune deficient state of the animal, injected human stem cells will be not recognised as foreign by the immune system and human blood cells will be produced in the mouse bone marrow after transplantation.

In NOD SCID mice, selective advantage of the ABCG2-transduced human stem cells will be investigated, although murine gp91*^{phox}* is functioning, which prevents the study of reconstitution in the oxidase activity. Our concrete experiments included two vector systems both of which may serve different therapeutic aim and comprising cDNA encoding ABCG2.

In one of the systems the half transporter of the ABC family was used, beside a first therapeutic gene, as a second gene which is a marker allowing in vivo or in vitro selection. The therapeutic gene inserted in a *cis* position, may be a transgene suitable for treatment of a hereditary disorder. The cDNA of the ABCG2 is inserted in a *trans* position. It is preferably provided that the ABCG2 protein is expressed only if the transgene is expressed as well. This vector was successfully applied as an example in cells modeling a form of the chronic granulomatosus disease. Variants of ABCG2 and related proteins of half transporters of the ABCG family may be appropriate for aiding the treatment of any hereditary disease.

A further aim of the inventors was to protect cells, e.g. bone marrow cells from cytotoxic drugs. There is no need in this case for a further therapeutic gene. Since minimization of a side reaction of e.g. the treatment of malignant disorders is the object in this case, e.g. the protection of healthy hematopoietic cells, a high expression level, which is achieved in the above examples, is advantageous.

In this work we have also demonstrated that the overexpression of the R482G ABCG2 protein efficiently protected the genetically modified progenitor cells against cytotoxic selection, allowing increased expression of the therapeutic gene. The mutant ABCG2 gave a specific protection against ADR, as compared to a similar drug-resistance provided by both ABCG2 variants against mitoxantrone. In mouse cell, to some respect the R482T mutant proved to be preferred. Overexpression of the ABCG2 protein did not interfere with granulocyte maturation or the functional correction of the differentiated effector cells.

### CITED DOCUMENTS

| |
|---|
| ALLIKMETS, R., L. SCHRIML, et al. (1998). A human placenta-specific ATP-binding cassette gene (ABCP) on chromosome 4q22 that is involved in multidrug resistance. Cancer Research 58: 5337-5339. |
| BABIOR, B. M., R. S. KIPNES AND J. T. CURNUTTE (1973). Biological defense mechanisms. The production by leukocytes of superoxide, a potential bactericidal agent. J Clin Invest 52, 741-744. |
| BATES, S. E., R. ROBEY, K. MIYAKE, K. RAO, D. D. ROSS AND T. LITMAN (2001). The role of half-transporters in multidrug resistance. J Bioenerg Biomembr 33, 503-511. |
| BAUM, C., S. HEGEWISCH-BECKER, et al. (1995). Novel retroviral vectors for efficient expression of the multidrug resistance (mdr-1) gene in early hematopoietic cells. J Virol 69(12): 7541-7. |
| BECKER, S., S. WASSER, et al. (1998). Correction of respiratory burst activity in X-linked chronic granulomatous cells to therapeutically relevant levels after gene transfer into bone marrow CD34+ cells. Hum Gene Ther 9(11): 1561-70. |
| BERGER, F., D. SOLIGO, et al. (2001). Efficient retrovirus-mediated transduction of primitive human peripheral blood progenitor cells in stroma-free suspension culture. Gene Ther 8(9): 687-96. |
| BUNTING, K. D. (2002). ABC transporters as phenotypic markers and functional regulators of stem cells. Stem Cells 20, 11-20. |
| BURRITT, J. B., G. N. FRITEL, I. DAHAN, E. PICK, D. ROOS AND A. J. JESAITIS (2000). Epitope identification for human neutrophil flavocytochrome b monoclonals 48 and 449. Eur J Haematol 65, 407-413. |
| CAVAZZANA-CALVO, M., S. HACEIN-BEY, et al. (2001). Gene therapy of severe combined immunodefciencies. J Gene Med 3(3): 201-6. |
| Doyle, L., W. Yang, et al.(1998). A multidrug resistance transporter from human MCF-7 breast cancer cells. Proc. Natl. Acad. Sci. USA 95: 15665-15670. |
| Dreesen, T.D., Johnson, D.H., and Henikoff, S. (1988) The brown protein of Drosophila melanogaster is similar to the white protein and to components of active transport complexes. Mol. Cell Biol. 8, 5206-5215. |
| FISCHER, A., A. W. SEGAL, et al. (1993). The management of chronic granulomatous disease. Eur J Pediatr 152(11): 896-9. |
| HAFKEMEYER, P., T. LICHT, et al. (2000). Chemoprotection of hematopoietic cells by a mutant P-glycoprotein resistant to a potent chemosensitizer of multidrug-resistant cancers. Hum Gene Ther 11(4): 555-65. |
| HAVENGA, M. J., A. B. WERNER, et al. (1998). Methotrexate selectable retroviral vectors for Gaucher disease. Gene Ther 5(10): 1379-88. |
| HONJO, Y., C. A. HRYCYNA, Q. W. YAN, W. Y. MEDINA-PEREZ, R. W. ROBEY, A. VAN DE LAAR, T. LITMAN, M. DEAN AND S. E. BATES (2001). Acquired mutations in the MXR/BCRP/ABCP gene alter substrate specificity in MXR/BCRP/ABCP-overexpressing cells. Cancer Res 61, 6635-6639. |
| Horton, R.M. (1993) "In vitro recombination and mutagenesis of DNA SOEing together tailor-made genes" In Methods in Molecular Biology, vol. 15 (PCR Protocols: Current Methods and Applications) (White, B. A., ed.), pp. 251-268, Humana Press, Totowa, NJ |
| HUA, T., T. HASEBE, A. SOMEYA, S. NAKAMURA, K. SUGIMOTO AND I. NAGAOKA (2000). Evaluation of the expression of NADPH oxidase components during maturation of HL-60 cells to neutrophil lineage. J Leukocyte Biol 68, 216-224. |
| ISHIZAWA, L., G. HANGOC, et al. (1993). Immunomagnetic separation of CD34+ cells from human bone marrow, cord blood, and mobilized peripheral blood. J Hematother 2(3): 333-8. |
| KIM, M., H. TURNQIST, J. JACKSON, M. SGAGIAS, Y. YAN, M. GONG, M. DEAN J. G. SHARP AND K. COWAN (2002). The multidrug resisitance transporter ABCG2 (breast cancer resistance protein 1) effluxes Hoechst 33342 and is overexpressed in haematopoietic stem cells) Clin Cancer Res 8, 22-28 |
| KINSELLA TM AND NOLAN GP. (1996). Episomal vectors rapidly and stably produce high-titer recombinant retrovirus. Hum Gene Ther. 7(12), 1405-13 |
| LICHT, T., F. HERRMANN, et al. (1997). In vivo drug-selectable genes: a new concept in gene therapy. Stem Cells 15(2): 104-11. |
| LICHT, T., S. K. GOLDENBERG, et al. (2000). Drug selection of MDR1-transduced hematopoietic cells ex vivo increases transgene expression and chemoresistance in reconstituted bone marrow in mice. Gene Ther 7(4): 348-58. |
| LITMAN, T., M. BRANGI, E. HUDSON, P. FETSCH, A. ABATI, D. D. ROSS, K. MIYAKE, J. H. RESAU AND S. E. BATES (2000). The multidrug-resistant phenotype associated with overexpression of the new ABC half-transporter, MXR (ABCG2). J Cell Sci 113, 2011-2021. |
| MALECH, H. L. (1999). Progress in gene therapy for chronic granulomatous disease. J Infect Dis 179 Suppl 2, S318-325. |
| MALECH, H. L., T. R. BAUER, JR., et al. (1997). Prospects for gene therapy of neutrophil defects. Semin Hematol 34(4): 355-61. |
| MALIEPAARD, M., G. L. SCHEFFER, et al. (2001). Subcellular localization and distribution of the breast cancer resistance protein transporter in normal human tissues. Cancer Res 61(8): 3458-64. |
| MILLER, A. D., J. V. GARCIA, et al. (1991). Construction and properties of retrovirus packaging cells based on gibbon ape leukemia virus. J Virol 65(5): 2220-4. |
| MIYAKE, K., L. MICKLEY, et al. (1999). Molecular cloning of cDNAs which are highly overexpressed in mitoxantrone-resistant cells: Demonstration of homology to ABC transport genes. Cancer Research 59: 8-13. |
| MULLER, M., E. BAKOS, E. WELKER, A. VARADI, U. A. GERMANN, M. M. GOTTESMAN, B. S. MORSE, I. B. RONINSON AND B. SARKADI (1996). Altered drug-stimulated ATPase activity in mutants of the human multidrug resistance protein. J Biol Chem 271, 1877-1883. |
| NAKAMURA, M., M. MURAKAMI, et al. (1987). Monoclonal antibody 7D5 raised to cytochrome b558 of human neutrophils: immunocytochemical detection of the antigen in peripheral phagocytes of normal subjects, patients with chronic granulomatous disease, and their carrier mothers. Blood 69(5): 1404-8. |
| NÉMET, K., G. FEKETE, et al. (1997). [Chronic granulomatous disease (CGD): dysfunction of the neutrophil granulocyte NADPH-oxidase enzyme system]. Orv Hetil 138(7): 397-401. |
| NÉMET, K., J. CERVENAK, et al. (2000). Improved transduction efficiency of gp91phox, a phagocyte NADPH oxidase subunit gene and LNGFR by incubation at 32 C posmansduction. J Gene Med 2: P189. |
| ÖZVEGY, C., A. VÁRADI, AND B. SARKADI (2002) Characterization of drug transport, ATP hydrolysis and nucleotide trapping by the human ABCG2 multidrug transporter: modulation of substrate specificity by a point mutation. J. Biol. Chem, submitted, under revision. |
| ÖZVEGY, C., T. LITMAN, et al. (2001). Functional characterization of the human multidrug transporter, ABCG2, expressed in insect cells. Biochem Biophys Res Commun 285(1): 111-7. |
| PEAR, W. S., G. P. NOLAN, et al. (1993). Production of high-titer helper-free retroviruses by transient transfection. Proc Natl Acad Sci U S A 90(18): 8392-6. |
| PEPLING, M., MOUNT, S. M. "Sequence of a cDNA from the Drosophila melanogaster white gene" (1990) Nucleic Acids Res. 18(6), 1633 |
| RABINDRAN, S. K., D. D. ROSS, L. A. DOYLE, W. YANG AND L. M. GREENBERGER (2000). Fumitremorgin C reverses multidrug resistance in cells transfected with the breast cancer resistance protein. Cancer Res 60, 47-50. |
| ROBBINS, P. D. (1997). Gene Therapy Protocols The Humana Press. |
| ROBEY, R. W., Y. HONJO, et al. (2001). A functional assay for detection of the mitoxantrone resistance protein, MXR (ABCG2). Biochim Biophys Acta 1512(2): 171-82. |
| ROCCHI, E., A. KHODJAKOV, et al. (2000). The product of the ABC half-transporter gene ABCG2 (BCRP/MXR/ABCP) is expressed in the plasma membrane. Biochem Biophys Res Commun 271(1): 42-6. |
| ROLFS, A., I. SCHULLER, et al. (1992). Quantification of PCR-product. PCR: Clinical diagnostics and research Berlin Heidelberg New York, Springer-Verlag: 201-206. |
| ROOS, D., M. DE BOER, et al. (1996). Mutations in the X-linked and autosomal recessive forms of chronic granulomatous disease. Blood 87(5): 1663-81. |
| ROSS, D. D., W. YANG, L. V. et al. (1999). Atypical multidrug resistance: breast cancer resistance protein messenger RNA expression in mitoxantrone-selected cell lines. J Natl Cancer Inst 91, 429-433. |
| SAULNIER, S. O., D. S. STEINHOFF, et al.(2000). Lentivirus-mediated gene transfe of gp91phox corrects chronic granulomatous disease phenotype in human X-CGD cells. J. Gene Med. 2(5). |
| SCHIEDLMEIER, B., A. J. SCHILZ, K. KUHLCKE, S. LAUFS, C. BAUM, W. J. ZELLER, H. G. ECKERT AND S. FRUEHAUF (2002). Multidrug resistance 1 gene transfer can confer chemoprotection to human peripheral blood progenitor cells engrafted in immunodeficient mice. Hum Gene Ther 13, 233-242. |
| SCHNELL, T., P. FOLEY, et al. (2001). Development of a self-inactivating, minimal lentivirus based on Simian immunodeficiency virus. Human Gene Ther. 11(3): 439-447. |
| SELLERS, S. E., J. F. TISDALE, B. A. AGRICOLA, M. E. METZGER, R. E. DONAHUE, C. E. DUNBAR AND B. P. SORRENTINO (2001). The effect of multidrug-resisitance 1 gene versus neo transduction on ex vivo and in vivo expansion of rhesus macaque hematopoietic repopulating cells. Blood 97, 1888-1890. |
| SMEETS, M. E., R. A. RAYMAKERS, G. VIERWINDEN, A. H. PENNINGS, H. WESSELS AND T. DE WITTE (1999). Triggering noncycling hematopoietic progenitors and leukemic blasts to proliferate increases anthracycline retention and toxicity by downregulating multidrug resistance. Blood 94, 2414-2423. |
| SOKOLIC, R. A., S. SEKHSARIA, Y. SUGIMOTO, N. WHITING-THEOBALD, G. F. LINTON, F. LI, M. M. GOTTESMAN AND H. L. MALECH (1996). A bicistronic retrovirus vector containing a picornavirus internal ribosome entry site allows for correction of X-linked CGD by selection for MDR1 expression. Blood 87, 42-50. |
| SZAKÁCS, G., ÖZVEGY, C., BAKOS, É., SARKADI, B, AND VÁRADI, A. (2001) Role of glycine-534 and glycine-1179 of human multidrug resistance protein (MDR1) in drug-mediated control of ATP hydrolysis. Biochem. J. 356, 71-75. |
| TEARLE, R. G., BELOTE, J. M., MCKEOWN, M., BAKER, B. S., HOWELLS, A. J. (1989) "Cloning and characterization of the scarlet gene of Drosophila melanogaster" Genetics 122(3) 595-606. |
| TRUCKER, K. A., M. B. LILLY, et al. (1987). Characterization of a new human diploid myeloid leukemia cell line (PLB-985) with granulocytic and monocytic differentiating capacity. Blood 70(2): 372-8. |
| TUCKER, K. A., M. B. LILLY, L. HECK, JR. AND T. A. RADO (1987). Characterization of a new human diploid myeloid leukemia cell line (PLB-985) with granulocytic and monocytic differentiating capacity. Blood 70,372-378. |
| VAN LOEVEZIJN, A., J. D. ALLEN, A. H. SCHINKEL AND G. J. KOOMEN (2001). Inhibition of BCRP-mediated drug efflux by fumitremorgin-type indolyl diketopiperazines. Bioorg Med Chem Lett 11, 29-32. |
| VERHOEVEN, A. J., B. G. BOLSCHER, et al. (1989). Characterization of two monoclonal antibodies against cytochrome b558 of human neutrophils. Blood 73(6): 1686-94. |
| WILLIAMS, D. A. AND F. O. SMITH (2000). Progress in the use of gene transfer methods to treat genetic blood diseases. Hum Gene Ther 11: 2059-2066. |
| YAMAUCHI, A., L. YU, A. J. POTGENS, F. KURIBAYASHI, H. NUNOI, S. KANEGASAKI, D. ROOS, H. L. MALECH, M. C. DINAUER AND M. NAKAMURA (2001). Location of the epitope for 7D5, a monoclonal antibody raised against human flavocytochrome b558, to the extracellular peptide portion of primate gp91phox. Microbiol Immunol 45, 249-257. |
| ZHEN, L., A. A. KING, et al. (1993). Gene targeting of X chromosome-linked chronic granulomatous disease locus in a human myeloid leukemia cell line and rescue by expression of recombinant gp91phox. Proc Natl Acad Sci U S A 90(21): 9832-6. |
| ZHOU, S., J. D. SCHUETZ, et al. (2001). The ABC transporter Bcrp1/ABCG2 is expressed in a wide variety of cells and is a molecular determinant of the side-population phenotype. Nature Med. 7(9): 1028-1034. |

### ABBREVIATIONS USED

MXR, Mitoxantrone Resistance-associated protein; BCRP, Breast Cancer Resistance Protein; ABCP, Placenta specific ABC transporter; ABC, ATP Binding Cassette; Sf9 cells, *Spodoptera frugiperda* ovarian cells; MDR1, Multidrug Resistance protein; MRP1, Multidrug Resistance-associated Protein; MX, mitoxantrone; FTC, Fumitremorgin C; CsA, cyclosporin A; CGD, chronic granulomatouse disease; gp91*^{phox}*, 91 kD glycoprotein, β-subunit of the phagocyte NADPH oxidase enzyme; PLB985 cell line, myelomonocytic cells; PLB985 X-CGD, gp91^{phox} knock-out version of the PLB985 cells; CFC colony forming cells; SOD; superoxide dismutase; DOX; doxorubicine

## Claims

1. Use of an isolated nucleic acid, preferably a cDNA, comprising a sequence encoding an ABCG2 half transporter protein as a selectable marker, in a method for selecting somatic mammalian cells *in vitro* by at least one drug transportable by the ABCG2 half transporter protein, provided that cells of human embryonic origin are excluded.

2. Use according to claim 1 wherein the somatic mammalian cells are selected from
- somatic pluripotent stem cells, e.g. bone marrow cells or hematopoietic progenitor cells, for example CD34+-cells or
- myeloid or lymphoid cells, for example monocytes, dendritic cells, T-cells, B-cells or NK-cells.

3. Use according to claim 1 or claim 2 wherein the ABCG2 half-transporter protein is an R482G mutant or an R482T mutant.

4. The use according to any of claims 1 to 3 wherein the sequence encoding an ABCG2 half-transporter protein is carried by a vector, wherein said vector is an expression vector useful for gene transfer into a mammalian cell.

5. The use according to claim 4 wherein the vector is a retroviral vector which comprises the nucleic acid sequence encoding the ABCG2 half-transporter protein as the only expressible marker between 5' and 3' LTR regions.

6. The use according to claim 4 or 5 wherein the vector is an expression vector useful for gene transfer into a mammalian cell, said vector comprising (a) the nucleic acid sequence encoding the ABCG2 half-transporter protein as a selectable marker, and (b) a further nucleotide sequence comprising the sequence of a transgene, and means for ensuring that expression of the two nucleotide sequences is operably linked.

7. The use according to claim 6, wherein the expression vector is a bicistronic viral vector and wherein the expression of both (a) the nucleic acid sequence encoding a ABCG2 half-transporter protein and (b) the nucleotide sequence of the transgene are controlled by a promoter operable in a mammalian cell, and
- the therapeutic gene is located in a *cis* position, the nucleotide sequence encoding the half-transporter protein in a *trans* position relative to the promoter, and
- between the two nucleotide sequences there is an SA site or an IRES site.

8. A nucleic acid having a nucleotide sequence encoding an ABCG2 half-transporter protein for use in gene therapy.

9. The nucleic acid for use according to claim 8, said use comprising transducing mammalian cells with a vector carrying the isolated nucleic acid of the ABCG2 half-transporter protein and selecting the cells by a cytotoxic drug transportable by the protein.

10. The nucleic acid for use according to claim 8 or claim 9 said use comprising a treatment with a transgene which is a therapeutic gene, wherein the cells carrying the transgene and the isolated nucleic acid are protected by the expressed half-transporter protein or selected by the cytotoxic drug.

11. The nucleic acid for use according to claim 10 said use comprising a transgenic treatment of an inherited immune deficiency disease, preferably chronic granulomatous disease (CGD) and the therapeutic gene is gp91^{phox}.

12. The nucleic acid for use according to any of claims 8 or 9 said use comprising a gene therapy intervention in diseases treated by a cytotoxic drug transportable by ABCG2, preferably in chemotherapy of tumorous diseases.

13. An expression vector for use in gene therapy, said vector being useful for gene transfer into a mammalian cell, and said vector comprising a nucleic acid sequence operably linked to a promoter operable in a mammalian cell and encoding an ABCG2 half-transporter protein as a selectable marker.

14. The vector for use according to claim 13 said use comprising protection of transformed mammalian cells of a patient against a cytotoxic drug.

15. The vector for use according to claim 13 said use comprising treatment of a genetic disorder said vector comprising, besides the nucleotide sequence encoding the half-transporter protein, a further nucleotide sequence comprising the sequence of a further transgene which is a therapeutic gene useful for treating the said genetic disorder.

16. The vector for use according to claim 14 or 15 which is a viral vector, preferably a retroviral vector, an oncovirus vector, a lentivirus vector, an adenovirus vector or an adeno associated virus vector.

17. The retroviral vector for use according to claim 16 said vector comprising a promoter operable in a mammalian cell, preferably a retroviral promoter, and means for ensuring that expression of the two nucleotide sequences is operably linked, wherein the expression is driven by a single promoter and
(a) the therapeutic gene is located in a *cis* position, the nucleotide sequence encoding the half-transporter protein in a *trans* position relative to the promoter,
(b) between the two nucleotide sequences there is an SA site or an IRES site.

18. The retroviral vector for use according to any of claims 16 to 17 wherein the mammalian cells are any of the cells as defined in claim 2.

19. The vector for use according to any of claims 13 to 18 comprising a nucleic acid sequence encoding a ABCG2 half-transporter protein as the only mammalian expressible marker.

20. A somatic mammalian cell provided that cells of human embryonic origin are excluded for use in gene therapy, said cell being transformed by the vector of any of claims 13 to 19.

21. A pharmaceutical kit for use in gene therapy, said kit comprising a vector of any of claims 13 to 19.

22. The pharmaceutical kit for use according to claim 21 wherein the vector is a viral vector and said kit optionally comprises one or more of the following: packaging cells applicable to the viral vector, means for further viral gene manipulation, buffers, media, cell lines and reagents.

23. A pharmaceutical composition comprising a vector of any of claims 13 to 19 for use in gene therapy.

24. A process for protecting somatic mammalian cells provided that cells of human embryonic origin are excluded for against a cytotoxic drug transportable by an ABCG2 half transporter protein said method comprising the steps of
i) treating mammalian cells with a vector, wherein said vector comprising a nucleotide sequence encoding the ABCG2 half-transporter protein and a further nucleotide sequence comprising the sequence of a transgene which is a therapeutic gene and thereby introducing the nucleotide sequence encoding the ABCG2 half-transporter into the cells,
ii) providing conditions appropriate for expression of the protein,
iii) exposing the mammalian cells to the cytotoxic drug, and
iv) selecting cells by the cytotoxic drug.

25. The vector for use of any of claims 13 to 19 for use in a method for gene transfer wherein cells contacted with a transgene are protected against a cytotoxic drug transportable by ABCG2 half transporter protein, said method comprising the steps of
i) obtaining somatic cells to be treated from a mammalian patient,
ii) treating the somatic cells with a vector of any of claims 13 to 19 and thereby introducing a nucleotide sequence encoding an ABCG2 half-transporter protein into the cells,
iii) reintroducing the treated cells into the patient,
iv) administering the cytotoxic drug to the patient, and
if desired, before or after reintroducing the treated cells, selecting the cells by a further cytotoxic drug, which can be the same as or different from the drug administered in step iv), transportable by the half transporter protein.

## Patentansprüche

1. Verwendung einer isolierten Nukleinsäure, vorzugsweise einer cDNA, die eine Sequenz umfasst, die ein ABCG2-Halbtransporterprotein als einen selektierbaren Marker kodiert, in einem Verfahren zum Invitro-Selektieren von somatischen Säugetierzellen durch Kultivieren der Zellen mit mindestens einem durch das ABCG2-Halbtransporterprotein transportierbaren Medikament, sofern Zellen menschlichen embryonalen Ursprungs ausgeschlossen sind.

2. Verwendung gemäß Anspruch 1, wobei die somatischen Säugetierzellen aus
- somatischen pluripotenten Stammzellen, zum Beispiel Knochenmarkszellen oder hämatopoetischen Vorläuferzellen, zum Beispiel CD34+-Zellen oder
- myeloiden oder lymphoiden Zellen, zum Beispiel Monozyten, dendritischen Zellen, T-Zellen, B-Zellen oder NK-Zellen
ausgewählt sind.

3. Verwendung gemäß Anspruch 1 oder Anspruch 2, wobei das ABCG2-Halbtransporterprotein ein R482G-Mutant oder ein R482T-Mutant ist.

4. Die Verwendung gemäß irgendeinem der Ansprüche 1 bis 3, wobei die ein ABCG2-Halbtransporterprotein kodierende Sequenz von einem Vektor getragen wird, wobei der Vektor ein Expressionsvektor ist, der für den Gentransfer in eine Säugetierzelle verwendbar ist.

5. Die Verwendung gemäß Anspruch 4, wobei der Vektor ein retroviraler Vektor ist, der die Nukleinsäuresequenz umfasst, die das ABCG2-Halbtransporterprotein als den einzigen exprimierbaren Marker zwischen 5 `- und 3 `-LTR-Bereichen kodiert.

6. Die Verwendung gemäß Anspruch 4 oder 5, wobei der Vektor ein Expressionsvektor ist, der für den Gentransfer in eine Säugetierzelle verwendbar ist, wobei der Vektor umfasst: (a) die Nukleinsäuresequenz, die das ABCG2-Halbtransporterprotein als einen selektierbaren Marker kodiert, und (b) eine weitere Nukleotidsequenz, die die Sequenz eines Transgens umfasst, und Mittel, um sicherzustellen, dass die Expression der beiden Nukleotidsequenzen in funktionsfähiger Weise miteinander verknüpft ist.

7. Die Verwendung gemäß Anspruch 6, wobei der Expressionsvektor ein bicistronischer viraler Vektor ist, und wobei die Expression sowohl (a) der Nukleinsäuresequenz, die ein ABCG2-Halbtransporterprotein kodiert, als auch (b) der Nukleotidsequenz des Transgens von einem Promotor gesteuert wird, der in einer Säugetierzelle funktionsfähig ist, und
- das therapeutische Gen in einer cis-Stellung angeordnet ist, wobei die Nukleotidsequenz das Halbtransporterprotein bezüglich des Promotors in einer trans-Stellung kodiert, und
- zwischen den beiden Nukleotidsequenzen eine SA-Stelle oder eine IRES-Stelle vorhanden ist.

8. Eine Nukleinsäure, die eine ein ABCG2-Halbtransporterprotein kodierende Nukleotidsequenz umfasst, zur Verwendung in der Gentherapie.

9. Die Nukleinsäure zur Verwendung gemäß Anspruch 8, wobei die Verwendung das Transduzieren von Säugetierzellen mit einem Vektor, der die isolierte Nukleinsäure des ABCG2-Halbtransporterproteins trägt, und das Selektieren der Zellen durch ein zytotoxisches Medikament, das durch das Protein transportierbar ist, umfasst.

10. Die Nukleinsäure zur Verwendung gemäß Anspruch 8 oder Anspruch 9, wobei die Verwendung eine Behandlung mit einem Transgen umfasst, welches ein therapeutisches Gen ist, wobei die das Transgen und die isolierte Nukleinsäure tragenden Zellen durch das exprimierte Halbtransporterprotein geschützt oder durch das zytotoxische Medikament selektiert werden.

11. Die Nukleinsäure zur Verwendung gemäß Anspruch 10, wobei die Verwendung eine transgene Behandlung einer erblichen Immundefekterkrankung, vorzugsweise der septischen Granulomatose (CGD) umfasst, und wobei das therapeutische Gen gp91^{phox} ist.

12. Die Nukleinsäure zur Verwendung gemäß irgendeinem der Ansprüche 8 oder 9, wobei die Verwendung eine Gentherapie-Maßnahme bei Krankheiten, die mittels eines durch ABCG2 transportierbaren zytotoxischen Medikaments behandelt werden, vorzugsweise bei der Chemotherapie von Tumorerkrankungen umfasst.

13. Ein Expressionsvektor zur Verwendung in der Gentherapie, wobei der Vektor für den Gentransfer in eine Säugetierzelle verwendbar ist, und wobei der Vektor eine Nukleinsäuresequenz umfasst, die funktionsfähig mit einem Promotor verbunden ist, der in einer Säugetierzelle funktioniert, und ein ABCG2-Halbtransporterprotein als einen auswählbaren Marker kodiert.

14. Der Vektor zur Verwendung gemäß Anspruch 13, wobei die Verwendung den Schutz von transformierten Säugetierzellen eines Patienten vor einem zytotoxischen Medikament umfasst.

15. Der Vektor zur Verwendung gemäß Anspruch 13, wobei die Verwendung die Behandlung eines Gendefekts umfasst, wobei der Vektor neben der das Halbtransporterprotein kodierenden Nukleotidsequenz eine weitere Nukleotidsequenz umfasst, die die Sequenz eines weiteren Transgens umfasst, das ein therapeutisches Gen ist, das zum Behandeln des Gendefekts verwendbar ist.

16. Der Vektor zur Verwendung gemäß Anspruch 14 oder 15, der ein viraler Vektor, vorzugsweise ein retroviraler Vektor, ein Onkovirusvektor, ein Lentivirusvektor, ein Adenovirusvektor oder ein Adenoassoziierter Virusvektor ist.

17. Der retrovirale Vektor zur Verwendung gemäß Anspruch 16, wobei der Vektor umfasst: einen Promotor, der in einer Säugetierzelle funktioniert, vorzugsweise einen retroviralen Promotor, und Mittel, um sicherzustellen, dass die Expression der beiden Nukleotidsequenzen funktional verknüpft ist, wobei die Expression durch einen einzigen Promotor angetrieben wird, und
(a) das therapeutische Gen in einer cis-Stellung angeordnet ist, die das Halbtransporterprotein kodierende Nukleotidsequenz bezüglich des Promotors in einer trans-Stellung angeordnet ist, und
(b) zwischen den beiden Nukleotidsequenzen eine SA-Stelle oder eine IRES-Stelle vorhanden ist.

18. Der retrovirale Vektor zur Verwendung gemäß irgendeinem der Ansprüche 16 bis 17, wobei die Säugetierzellen irgendwelche der wie in Anspruch 2 definierten Zellen sind.

19. Der Vektor zur Verwendung gemäß irgendeinem der Ansprüche 13 bis 18, umfassend eine Nukleinsäuresequenz, die ein ABCG2-Halbtransporterprotein als den einzigen Säugetier-exprimierbaren Marker kodiert.

20. Eine somatische Säugetierzelle, wobei vorausgesetzt wird, dass Zellen menschlichen embryonalen Ursprungs ausgeschlossen sind, zur Verwendung in der Gentherapie, wobei die Zelle durch den Vektor gemäß irgendeinem der Ansprüche 13 bis 19 transformiert wird.

21. Ein pharmazeutisches Kit zur Verwendung in der Gentherapie, wobei das Kit einen Vektor gemäß irgendeinem der Ansprüche 13 bis 19 umfasst.

22. Das pharmazeutische Kit zur Verwendung gemäß Anspruch 21, wobei der Vektor ein viraler Vektor ist und das Kit optional eines oder mehrere der folgenden umfasst: Verpackungszellen, die für den viralen Vektor anwendbar sind, Mittel für eine weitere virale Genmanipulation, Puffer, Medien, Zelllinien und Reagenzien.

23. Eine pharmazeutische Zusammensetzung, die einen Vektor gemäß irgendeinem der Ansprüche 13 bis 19 umfasst, zur Verwendung in der Gentherapie.

24. Ein Verfahren zum Schützen somatischer Säugetierzellen, wobei vorausgesetzt wird, dass Zellen menschlichen embryonalen Ursprungs ausgeschlossen sind, vor einem zytotoxischen Medikament, das durch ein ABCG2-Halbtransporterprotein transportierbar ist, wobei das Verfahren die folgenden Schritte umfasst:
i) Behandeln von Säugetierzellen mit einem Vektor, wobei der Vektor eine Nukleotidsequenz, die das ABCG2-Halbtransporterprotein kodiert, und eine weitere Nukleotidsequenz umfasst, die die Sequenz eines Transgens umfasst, welches ein therapeutisches Gen ist, und dadurch Einbringen der Nukleotidsequenz, die den ABCG2-Halbtransporter kodiert, in die Zellen,
ii) Bereitstellen von Bedingungen, die für die Expression des Proteins geeignet sind,
iii) Aussetzen der Säugetierzellen dem zytotoxischen Medikament, und
iv) Selektieren von Zellen durch das zytotoxische Medikament.

25. Der Vektor zur Verwendung gemäß irgendeinem der Ansprüche 13 bis 19 zur Verwendung in einem Gentransferverfahren, wobei mit einem Transgen kontaktierte Zellen vor einem zytotoxischen Medikament geschützt werden, das durch ein ABCG2-Halbtransporterprotein transportierbar ist, wobei das Verfahren die folgenden Schritte umfasst:
i) Gewinnen von zu behandelnden somatischen Zellen aus einem Säugetierpatienten,
ii) Behandeln der somatischen Zellen mit einem Vektor gemäß irgendeinem der Ansprüche 13 bis 19, und dadurch Einbringen einer ein ABCG2-Halbtransporterprotein kodierenden Nukleotidsequenz in die Zellen,
iii) erneutes Einbringen der behandelten Zellen in den Patienten,
iv) Verabreichen des zytotoxischen Medikaments an den Patienten, und,
falls gewünscht, vor oder nach dem erneuten Einbringen der behandelten Zellen Selektieren der Zellen durch ein weiteres zytotoxisches Medikament, welches das gleiche oder ein Medikament sein kann, das sich von dem in Schritt iv) verabreichten Medikament unterscheidet, das durch das Halbtransporterprotein transportierbar ist.

## Revendications

1. Utilisation d'un acide nucléique isolé, de préférence un ADNc, comprenant une séquence codant une protéine demi-transporteur ABCG2 en tant que marqueur sélectionnable, dans un procédé de sélection *in vitro* de cellules somatiques de mammifère par la culture desdites cellules avec au moins un médicament pouvant être transporté par la protéine demi-transporkeur ABCG2, à condition que les cellules d'origine embryonnaire humaine soient exclues.

2. Utilisation selon la revendication 1, dans laquelle les cellules somatiques de mammifère sont choisies parmi :
- les cellules souches somatiques pluripotentes, par exemple des cellules de moelle osseuse ou des cellules progénitrices hématopoïétiques, par exemple des cellules CD34+, ou
- les cellules myéloïdes ou lymphoïdes, par exemple les monocytes, les cellules dendritiques, les lymphocytes T, les lymphocytes B ou les lymphocytes tueurs naturels.

3. Utilisation selon la revendication 1 ou la revendication 2, dans laquelle la protéine demi-transporteur ABCG2 est un mutant R482G ou un mutant R482T.

4. Utilisation selon l'une quelconque des revendications 1 à 3, dans laquelle la séquence codant une protéine demi-transporteur ABCG2 est portée par un vecteur, ledit vecteur étant un vecteur d'expression utile pour transférer un gène dans une cellule de mammifère.

5. Utilisation selon la revendication 4, dans laquelle le vecteur est un vecteur rétroviral comprenant la séquence d'acide nucléique codant la protéine demi-transporteur ABCG2 en tant qu'unique marqueur exprimable entre les régions LTR 5' et 3'.

6. Utilisation selon la revendication 4 ou 5, dans laquelle le vecteur est un vecteur d'expression utile pour transférer un gène dans une cellule de mammifère, ledit vecteur comprenant (a) la séquence d'acide nucléique codant la protéine demi-transporteur ABCG2 en tant que marqueur sélectionnable, et (b) une séquence nucléotidique supplémentaire comprenant la séquence d'un transgène, et un moyen pour assurer que l'expression des deux séquences nucléotidiques est fonctionnellement liée.

7. Utilisation selon la revendication 6, dans laquelle le vecteur d'expression est un vecteur viral bicistronique et dans laquelle l'expression (a) de la séquence d'acides nucléiques codant une protéine demi-transporteur ABCG2 et (b) de la séquence nucléotidique du transgène est régulée par un promoteur utilisable dans une cellule de mammifère, et
le gène thérapeutique est situé en position *cis,* la séquence nucléotidique codant la protéine demi-transporteur étant en position *trans* par rapport au promoteur, et
entre les deux séquences nucléotidiques se trouve un site SA ou un site IRES.

8. Acide nucléique possédant une séquence nucléotidique codant une protéine demi-transporteur ABCG2, pour une utilisation en thérapie génique.

9. Acide nucléique pour une utilisation selon la revendication 8, ladite utilisation comprenant la transduction de cellules de mammifère avec un vecteur portant l'acide nucléique isolé de la protéine demi-transporteur ABCG2 et la sélection des cellules par un médicament cytotoxique pouvant être transporté par la protéine.

10. Acide nucléique pour une utilisation selon la revendication 8 ou la revendication 9, ladite utilisation comprenant un traitement avec un transgène qui est un gène thérapeutique, dans laquelle les cellules portant le transgène et l'acide nucléique isolé sont protégées par la protéine demi-transporteur exprimée ou sélectionnées par le médicament cytotoxique.

11. Acide nucléique pour une utilisation selon la revendication 10, ladite utilisation comprenant le traitement transgénique d'une maladie héréditaire de déficience immunitaire, de préférence une granulomatose chronique familiale (CGD) et le gène thérapeutique est gp91^{phox}.

12. Acide nucléique pour une utilisation selon la revendication 8 ou la revendication 9, ladite utilisation comprenant une intervention par thérapie génique sur des maladies traitées par un médicament cytotoxique pouvant être transporté par ABCG2, de préférence dans la chimiothérapie des maladies tumorales.

13. Vecteur d'expression pour une utilisation en thérapie génique, ledit vecteur étant utile pour transférer un gène dans une cellule de mammifère, et ledit vecteur comprenant une séquence d'acides nucléiques fonctionnellement liée à un promoteur utilisable dans une cellule de mammifère et codant une protéine demi-transporteur ABCG2 en tant que marqueur sélectionnable.

14. Vecteur pour une utilisation selon la revendication 13, ladite utilisation comprenant la protection de cellules transformées de mammifère issues d'un patient contre un médicament cytotoxique.

15. Vecteur pour une utilisation selon la revendication 13, ladite utilisation comprenant le traitement d'un trouble génétique, ledit vecteur comprenant, en plus de la séquence nucléotidique codant la protéine demi-transporteur, une autre séquence nucléotidique comprenant la séquence d'un autre transgène qui est un gène thérapeutique utile pour traiter ledit trouble génétique.

16. Vecteur pour une utilisation selon la revendication 14 ou 15, qui est un vecteur viral, de préférence un vecteur rétroviral, un vecteur oncovirale, un vecteur lentiviral, un vecteur adénoviral ou un vecteur viral adéno-associé.

17. Vecteur rétroviral pour une utilisation selon la revendication 16, ledit vecteur comprenant un promoteur utilisable dans une cellule de mammifère, de préférence un promoteur rétroviral, et un moyen pour assurer que l'expression des deux séquences nucléotidiques est fonctionnellement liée, dans laquelle l'expression est dirigée par un unique promoteur, et
(a) le gène thérapeutique est situé en position *cis,* la séquence nucléotidique codant la protéine demi-transporteur étant en position *trans* par rapport au promoteur,
(b) entre les deux séquences nucléotidiques se trouve un site SA ou un site IRES.

18. Vecteur rétroviral pour une utilisation selon la revendication 16 ou la revendication 17, dans lequel les cellules de mammifère sont l'une quelconque des cellules définies dans la revendication 2.

19. Vecteur pour une utilisation selon l'une quelconque des revendications 13 à 18, comprenant une séquence d'acides nucléiques codant une protéine demi-transporteur ABCG2 en tant qu'unique marqueur mammifère exprimable.

20. Cellule somatique de mammifère, à condition que les cellules d'origine embryonnaire humaine soient exclues, pour une utilisation en thérapie génique, ladite cellule étant transformée par le vecteur selon l'une quelconque des revendications 13 à 19.

21. Kit pharmaceutique pour une utilisation en thérapie génique, ledit kit comprenant un vecteur selon l'une quelconque des revendications 13 à 19.

22. Kit pharmaceutique pour une utilisation selon la revendication 21, dans lequel le vecteur est un vecteur viral et ledit kit comprend facultativement un ou plusieurs des éléments suivants : des cellules d'encapsidation applicables au vecteur viral, des moyens pour une autre manipulation génétique virale, des tampons, des milieux, des lignées cellulaires et des réactifs.

23. Composition pharmaceutique comprenant un vecteur selon l'une quelconque des revendications 13 à 19, pour une utilisation en thérapie génique.

24. Procédé de protection de cellules somatiques de mammifère, à condition que les cellules d'origine embryonnaire humaine soient exclues, contre un médicament cytotoxique pouvant être transporté par une protéine demi-transporteur ABCG2, ledit procédé comprenant les étapes suivantes :
i) le traitement de cellules de mammifère avec un vecteur, ledit vecteur comprenant une séquence nucléotidique codant la protéine demi-transporteur ABCG2 et une autre séquence nucléotidique comprenant la séquence d'un transgène qui est un gène thérapeutique, pour ainsi introduire la séquence nucléotidique codant le demi-transporteur ABCG2 dans les cellules,
ii) l'apport de conditions appropriées pour l'expression de la protéine,
iii) l'exposition des cellules de mammifère au médicament cytotoxique, et
iv) la sélection des cellules par le médicament cytotoxique.

25. Vecteur pour une utilisation selon l'une quelconque des revendications 13 à 19, pour une utilisation dans un procédé de transfert de gène, dans laquelle les cellules mises en contact avec un transgène sont protégées contre un médicament cytotoxique pouvant être transporté par une protéine demi-transporteur ABCG2, ledit procédé comprenant les étapes suivantes :
i) l'obtention de cellules somatiques à traiter provenant d'un patient mammifère,
ii) le traitement des cellules somatiques avec un vecteur selon l'une quelconque des revendications 13 à 19, pour ainsi introduire une séquence nucléotidique codant une protéine demi-transporteur ABCG2 dans les cellules,
iii) la réintroduction des cellules traitées dans le patient,
iv) l'administration du médicament cytotoxique au patient, et
si cela est souhaité, avant ou après la réintroduction des cellules traitées, la sélection des cellules par un autre médicament cytotoxique, qui peut être identique au médicament administré dans l'étape iv) ou différent de celui-ci, transportable par la protéine demi-transporteur.
